# EUROPEAN PATENT APPLICATION

(11) **EP 1 970 064 A1**
(43) Date of publication of application: **17.09.2008**
(21) Application number: 07090043.6
(22) Date of filing: 12.03.2007
(51) Int. Cl.: A61K 31/695, C07F 7/02, C07K 7/06

(54) **Silicon-derivatives for pet-imaging**

(71) Applicant: Bayer Schering Pharma Aktiengesellschaft, 13353 Berlin (DE)
(72) Inventor: Srinivasan, Ananth, 10629 Berlin (DE); Klar, Ulrich, 13503 Berlin (DE); Lehmann, Lutz, 13357 Berlin (DE); Voigtmann, Ulrike, 10115 Berlin (DE); Stellfeld, Timo, 14197 Berlin (DE); Höhne, Aileen, 8049 Zürich (CH); Mu, Linjing, 4123 Allschwil (CH); Ametamey, Simon, 8045 Zürich (CH)

(57) **Abstract**

This invention relates to silicon compounds suitable for labelling or already labelled with ¹⁸F, methods of preparing such a compound, compositions comprising such compounds, kits comprising such compounds or compositions and uses of such compounds, compositions or kits for diagnostic imaging, preferably positron emission tomography (PET).

## Description

### Field of Invention

This invention relates to novel compounds suitable for labelling or already labelled with ¹⁸F, methods of preparing such a compound, compositions comprising such compounds, kits comprising such compounds or compositions and uses of such compounds, compositions or kits for diagnostic imaging, preferably positron emission tomography (PET).

### Background Art

Molecular imaging has the potential to detect disease progression or therapeutic effectiveness earlier than most conventional methods in the fields of oncology, neurology and cardiology. Of the several promising molecular imaging technologies having been developed as optical imaging and MRI, PET is of particular interest for drug development because of its high sensitivity and ability to provide quantitative and kinetic data.

Positron emitting isotopes include carbon, nitrogen, and oxygen. These isotopes can replace their non-radioactive counterparts in target compounds to produce tracers that function biologically and are chemically identical to the original molecules for PET imaging. On the other hand, ¹⁸F is the most convenient labelling isotope due to its relatively long half life (109.6 min) which permits the preparation of diagnostic tracers and subsequent study of biochemical processes. In addition, its low ß+ energy (635 keV) is also advantageous.

Recently, Coenen published a review article describing chemical and technical aspects of ¹⁸F-labeling (Fluorine-18 Labelling Methods: Features and Possibilities of Basic Reactions; PET chemistry - the driving force in molecular imaging, Springer, 2006 in press): "[¹⁸F]fluoride is obtained as an aqueous solution [...]. Due to the high charge density of the anion it is strongly hydrated (ΔHhydr = 506 kJ/mol) and inactivated for nucleophilic reactions. [...] It is very easily protonated, forming hydrogen fluoride (EB = 565 kJ/mol), which then makes it unavailable for further reactions. Labelling, therefore, has to take place under aprotic but polar conditions. [...] However, routinely the nucleophilic radiofluorination with appropriate precursors is performed in dipolar aprotic solvents using fluoride salts with a soft cation (Cs+, Rb+) thus rendering weak - easy to separate - ion pairs and 'naked' [¹⁸F]fluoride of high nucleophilicity. For further anion activation phase transfer catalysts (PTC) like tetraalkylammonium carbonates (hydrogen carbonates) [...] or mainly the aminopolyether Kryptofix® 2.2.2 in combination with potassium carbonate or oxalate [...] are optimal. [...] Since fluoride in dipolar aprotic solvents exhibits also a strong basic character and generally the reaction medium is basic (CO₃ ²⁻ , HCO₃⁻ ), elimination reactions can complete with nucleophilic substitution. [...] A problem is the basicity of the reaction solution. Thus, base labile compounds such as butyrophenone neuroleptics, for example, could only be ¹⁸F-labelled directly when the [K⊂ 2.2.2]₂CO₃/C₂O₄ buffer system was used [...]." For these reasons it would be desirable to have a method which allow the ¹⁸F radiolabelling when acid is added; in particular the ¹⁸F radiolabeling of base-labile compounds which.would profit from such a method.

PET tracers are or often include a molecule of biological interest. Biomolecules developed for use in PET have been numerous intended for specific targeting in the patient as e.g. FDG, FLT, L-DOPA, methionine and deoxythymidine. Due to their specific use, such biomolecules are often designated as "targeting agents".

Several approaches for incorporating ¹⁸F in more complex biomolecules as e.g. peptides are described in the following references: European J. Nucl. Med Mol. Imaging 2001, 28, 929-938; European J. Nucl. Med Mol. Imaging 2004, 31, 1182-1206; Bioconjugate Chem. 1991, 2, 44-49; Bioconjugate Chem. 2003, 14, 1253-1259.

These methods are indirect. They demand at least a two step procedure for tracer synthesis. Therefore they are time consuming thereby reducing PET image resolution as a result of nuclear decay.

Very recently, Schirrmacher et al. described the preparation of ¹⁸F-organofluorsilanes via ¹⁸F-¹⁹F isotope exchange reactions. A ¹⁸F-Tyr3-octreotate derivative used for diagnosis of neuroendocrine tumors was synthesized (Angew. Chem. 2006, 118, 6193-6197). Nevertheless, this tracer prepared via isotope exchange reactions contains predominantly the corresponding non-radioactive ¹⁹F-compound which leads to products with relatively low specific activity. The use of other leaving groups followed by an efficient separation of the non-radioactive precursor would therefore be preferred.

Object of the present invention is therefore the development of a practical and mild technique for radio-labelling, in particular ¹⁸F labelling, of targeting agents like peptides in only one rather than two or more chemical steps in order to save time, costs and additional purification steps of radioactive compounds.

### Summary of the Invention

The object of the present invention is solved as detailed below.

In the first place, the present invention provides novel compounds represented by formula I 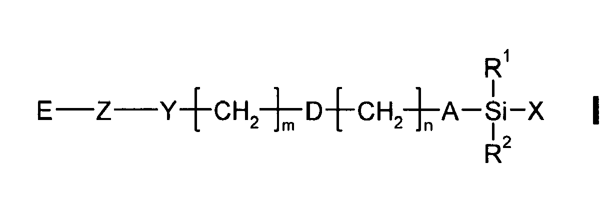 wherein
X represents a leaving group suitable for fluorination, selected from hydrogen or OR³, wherein R³ represents hydrogen, C₂-C₂₀ alkyl, C₂-C₁₀ alkenyl, C₂-C₁₀ alkynyl, aryl, heteroaryl or aralkyl;
R¹ and R², independently, represent hydrogen, C₁-C₁₀ alkyl, aryl, heteroaryl or aralkyl;
A represents a bond, or aryl, substituted aryl or heteroaryl
m and n, independently, can be any integer between 0 to 5;
D represents a bond, a sulphur atom, an oxygen atom or NR⁴,
wherein R⁴ represents hydrogen, C₁-C₁₀ alkyl, aryl, heteroaryl or aralkyl;
E-Z-Y represents a moiety selected from EO-C(=O), ENR⁵-C(=O), EC(=O)-O, EC(=O)-NR⁵, ENR⁵-SO₂, ESO₂-NR⁵, E-O, E-(S)p, E-NR⁵, ENR⁶C(=O)-NR⁷, ENR⁶-C(=O)NR⁷, ENR⁶C(=S)-NR⁷ , ENR⁶-C(=S)NR⁷, EO-C(=O)O, EOC(=O)-O, EOC(=S)-O, EO-C(=S)O (wherein the single bond explicitly shown in the formulae is the bond between Z and Y) and 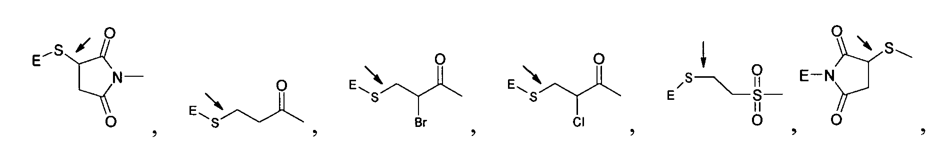 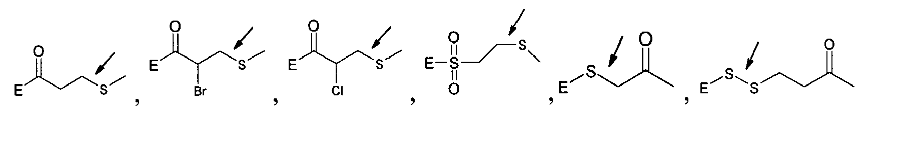 (wherein the arrow indicates the bond between Z and Y),
wherein R⁵, R⁶ and R⁷, independently, represent hydrogen, C₁-C₁₀ alkyl, aryl, heteroaryl or aralkyl, and
p can be any integer between 1 and 3; and
E-Z is a targeting agent radical.

Preferably, the leaving group X is selected from the group consisting of hydrogen, hydroxy, (C₂-C₂₀)alkoxy, C₂-C₁₀ alkynyloxy, and ar(C₁-C₂₀)alkoxy.

More preferably, the leaving group X is selected from the group consisting of hydrogen, hydroxy, (C₆-C₂₀)alkoxy, C₆-C₁₀ alkynyloxy and ar(C₆-C₂₀)alkoxy.

Even more preferably, the leaving group X is selected from the group consisting of hydrogen, hydroxy and ar(C₆-C₂₀)alkoxy.

Especially preferred is the leaving group X selected from the group consisting of (C₆-C₂₀)alkoxy, C₆-C₁₀ alkynyloxy and ar(C₆-C₂₀)alkoxy.

Most preferably the leaving group X is selected from the group consisting of hydrogen and hydroxyl.

Advantageously, R¹ and R², independently, are branched C₂-C₅ alkyl groups.

Most preferably, R¹ and R² are isopropyl, tert-butyl or isobutyl.

Preferably, the targeting agent is selected from peptides, small molecules or oligonucleotides.

More preferably, the targeting agent is -NR⁴-peptide or -NR⁴-(CH₂)ₙ- peptide, R⁴ being independently selected from hydrogen or alkyl, wherein n is between 1 and 6.

Most preferably, the targeting agent is a peptide comprising from 4 to 100 amino acids.

Furthermore, the invention refers to novel compounds represented by formula II 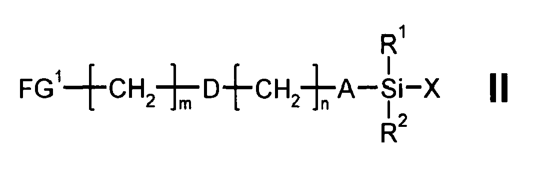 wherein
FG¹ represents OH, Hal, N₃, CO₂R⁸, NHR⁵, N=C=O, N=C=S, O-SO₂-Aryl, OSO₂-Alkyl, SO₂-Hal, S₃H, SH, O-C(=O)-Hal, O-C(=S)-Hal, 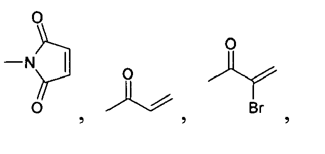 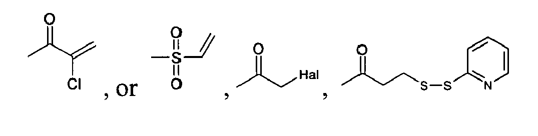 wherein Hal represents a halogen atom, and
R⁸ represents hydrogen, C₁-C₁₀ alkyl, C₂-C₁₀ alkenyl, aralkyl, or 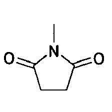 and wherein X, R¹, R², A, D, m, and n have the same meanings as in formula I.

Preferred compounds of formula II are
(Di-tert-butyl-hydroxy-silanyl)-acetic acid
[4-(Hydroxy-diisopropyl-silanyl)-phenyl]-acetic acid
[4-(Hydroxy-diisopropyl-silanyl)-phenyl]-acetic acid
(4-Diisopropylsilanyl-phenyl)-acetic acid
(4-Diisopropylsilanyl-phenyl)-acetic acid 2,5-dioxo-pyrrolidin-1-yl ester
[4-(Hydroxy-diisopropyl-silanyl)-phenyl]-acetic acid 2,5-dioxo-pyrrolidin-1-yl ester
4-Diisopropylsilanyl-benzoic acid
4-(Hydroxy-diisopropyl-silanyl)-benzoic acid
4-Diisopropylsilanyl-benzoic acid 2,5-dioxo-pyrrolidin-1-yl ester
4-(Hydroxy-diisopropyl-silanyl)-benzoic acid 2,5-dioxo-pyrrolidin-1-yl ester
3-(4-Diisopropylsilanyl-phenyl)-propionic acid
3-(4-Diisopropylsilanyl-phenyl)-propionic acid 2,5-dioxo-pyrrolidin-1-yl ester
3-(3-Diisopropylsilanyl-phenyl)-propionic acid
3-(3-Diisopropylsilanyl-phenyl)-propionic acid 2,5-dioxo-pyrrolidin-1-yl ester
(3-Diisopropylsilanyl-phenyl)-acetic acid
(3-Diisopropylsilanyl-phenyl)-acetic acid 2,5-dioxo-pyrrolidin-1-yl ester
(4-Diisobutylsilanyl-phenyl)-acetic acid
(4-Diisobutylsilanyl-phenyl)-acetic acid 2,5-dioxo-pyrrolidin-1-yl ester
[4-(Hydroxy-diisobutyl-silanyl)-phenyl]-acetic acid 2,5-dioxo-pyrrolidin-1-yl ester
[4-(Hydroxy-diisobutyl-silanyl)-phenyl]-acetic acid
4-Diisobutylsilanyl-benzoic acid
4-(Hydroxy-diisobutyl-silanyl)-benzoic acid
4-Diisobutylsilanyl-benzoic acid 2,5-dioxo-pyrrolidin-1-yl ester
4-(Hydroxy-diisobutyl-silanyl)-benzoic acid 2,5-dioxo-pyrrolidin-1-yl ester
4-[3-(Ethoxy-diisopropyl-silanyl)-propylcarbamoyl]-butyric acid
4-[3-(Ethoxy-diisopropyl-silanyl)-propylcarbamoyl]-butyric acid 2,5-dioxo-pyrrolidin-1-yl ester
5-[Diisobutyl-(4-phenyl-butoxy)-silanyl]-pentanoic acid 2,5-dioxo-pyrrolidin-1-yl ester
5-[(4-Polystyrene-methoxy-benzyloxy)-diisobutyl-silanyl]-pentanoic acid 2,5-dioxo-pyrrolidin-1-yl ester
5-(Polystyrene-methoxy-diisobutyl-silanyl)-pentanoic acid 2,5-dioxo-pyrrolidin-1-yl ester and
5-(Polystyrene-ethoxy-diisobutyl-silanyl)-pentanoic acid 2,5-dioxo-pyrrolidin-1-yl ester

The present invention is also related to a method for producing a compound according to general formula I, as defined herein above, wherein a compound of general formula II, as also defined herein above, is reacted with a compound of general formula III

E-FG² III

wherein
FG² has the meanings as listed for FG¹, and E has the same meaning as defined herein above, and wherein FG¹ and FG² are selected to establish Z-Y, as defined herein above.

The present invention furthermore relates to a method for producing a compound of general formula I or of general formula II, as defined herein above, wherein X is ¹⁸F, by reacting a compound of general formula I or II, respectively, wherein X is different from ¹⁸F, with a ¹⁸F fluorination agent under known conditions and/or under a novel condition.

"Known conditions" - as mentioned above - comprises but are not limited to those radiofluorination reactions which are carried out, for example in a typical reaction vessel (e.g. Wheaton vial) being known to someone skilled in the art or in a microreactor. The reaction can be heated by typical methods, e.g. oil bath, heating block or microwave.

The radiofluorination reactions can be carried out in dimethylformamide with potassium carbonate as base and "kryptofix" as crown-ether. But also other solvents can be used which are well known to experts. These possible conditions include, but are not limited to: dimethylsulfoxid and acetonitril as solvent and tetraalkyl ammonium and tertraalkyl phosphonium carbonate as base. Water and/or alcohol can be involved in such a reaction as co-solvent. The radiofluorination reactions are conducted for 1 to 45 minutes. Preferred reaction times are 3 to 40 minutes. Further preferred reaction times are 5 to 30 min."

This novel condition comprises the use of inorganic acid and/or organic acid in the ¹⁸F radiolabeling reaction.

Preferably organic acids are used in the ¹⁸F radiolabeling reaction.

More preferably aliphatic, cycloaliphatic, aromatic, araliphatic, heterocyclic carboxylic and sulphonic acids are used.in the ¹⁸F radiolabeling reaction.

Most preferably aliphatic carboxylic acids are used, including but not limited to propionic acid, acetic acid and formic acid.

Preferably, the reaction temperature is 80°C or less.

Most preferably, the reaction temperature is 50°C or less.

Compounds obtainable by this method are, for example,
[4-(Fluoro-diisopropyl-silanyl)-phenyl]-acetic acid
2-[4-(Fluoro-diisopropyl-silanyl)-phenyl]-ethanol
4-(Fluoro-diisopropyl-silanyl)-benzoic acid
[4-(Fluoro-diisopropyl-silanyl)-phenyl]-methanol
3-[4-(Fluoro-diisopropyl-silanyl)-phenyl]-propan-1-ol
3-[4-(Fluoro-diisopropyl-silanyl)-phenyl]-propionic acid
3-[3-(Fluoro-diisopropyl-silanyl)-phenyl]-propan-1-ol
3-[3-(Fluoro-diisopropyl-silanyl)-phenyl]-propionic acid
2-[3-(Fluoro-diisopropyl-silanyl)-phenyl]-ethanol
[3-(Fluoro-diisopropyl-silanyl)-phenyl]-acetic acid
[4-(Fluoro-diisobutyl-silanyl)-phenyl]-acetic acid and
4-(Fluoro-diisobutyl-silanyl)-benzoic acid
wherein fluoro means ¹⁸F.

Also, the present invention relates to a composition, further comprising a physiologically acceptable diluent or carrier.

Moreover, the present invention relates to a kit comprising a compound or composition, as defined herein above, in powder form, and a container containing an appropriate solvent for preparing a solution of the compound or composition for administration to an animal, including a human.

Finally, the invention relates to the use of a compound, composition or kit, as defined herein above, for diagnostic imaging.

Preferably, the use is for positron emission tomography.

Most preferably, the use is for imaging of tumors, imaging of inflammatory and/or neurodegenerative diseases, such as multiple sclerosis or Alzheimer's disease, or imaging of angiogenesis-associated diseases, such as growth of solid tumors, and rheumatoid arthritis.

Embodiments of this invention include methods involving the ¹⁸F fluorination of compounds ready for use as imaging agents and the ¹⁸F containing compounds derived from them. The compounds subjected to fluorination may already include a targeting agent for imaging purposes. Preferred embodiments of this invention involve the formation of a precursor molecule, which may include a targeting agent, prior to fluorination with ¹⁸F being the last step in the process prior to preparation of the compound for administration to an animal, in particular a human.

The use of silicon derivatives described herein facilitates the process. Thus, a desired PET imaging agent may be proposed starting from a silicon derivative which is then subjected to ¹⁸F fluorination.

Substituents on such silicon derivatives include linking groups or reactive groups designed for subsequent addition of a targeting agent. Linking groups may include aliphatic or aromatic molecules and readily form a bond to a selected, appropriate functionalized targeting agent. A variety of such groups is known in the art. These include carboxylic acids, carboxylic acid chlorides and active esters, sulfonic acids, sulfonyl-chlorides amines, hydroxides, thiols etc. on either side.

Contemplated herein are also groups which provide for ionic, hydrophobic and other non-convalent bonds between silicon derivative and targeting agent.

A few publications are known which describe the preparation of silicon based ¹⁸F-labelled tracers:

Rosenthal et al. published the first report on ¹⁸F labelling of silyl derivatives. ¹⁸F-Fluorotrimethylsilane was prepared by reaction of TMAF with chlorotrimethylsilane in aqeous acetontrile. Rats which were allowed to inhale the gas showed an extensive uptake of ¹⁸F in the bone, demonstrating a fast release of fluoride in vivo. (Int. J. Appl. Radiat. Isot. 1985, 36 (4), 318-319.)

Walsh et al. described a PET model compound containing one ¹⁸F, two phenyl groups and a tertiary butyl group attached to silicon with sufficient hydrolytic stability. Based on this model compound the inclusion of a thio-reactive or amine-reactive group for subsequent attached to a targeting agent was planned according to the abstract, but to our knowdlege not published yet. (J. Labelled Cpd. Radiopharm. 1999, 42 Suppl.1, S1-S3; J. Nuclear Medicine, Supp. S. 2000, 41, 1098.) Nevertheless, such an indirect approach needs a least two synthetic steps, labelling and binding to a targeting agent to get a useful PET probe.

Perrin et al. described that multiple F atoms may be joined to silicon to incorporate a greater number of ¹⁸F atoms into a single tracer. This enhances the density of positron emitters in the resulting product and stabilizes the silicon moiety under physiological conditions with a rate that is on par with that of ¹⁸F decay. But, depending on the number of fluorine atoms incorporated, compounds of this invention may be charged or uncharged which is not predictable leading to mixtures of products. (J. Am. Chem. Soc. 2005, 127, 13094-13095. WO2005077967)

In general, the preparation of silyl fluorides is well understood in the art especially in the cleavage of trialkyl silyl hydroxy protecting groups by TBAF, HF or KF which results in trialkyl silyl fluorides. (Greene et al. "Protective Groups in Organic Synthesis", Third Edition, 1999, Wiley VCH.)

Sieburth et al. described the conversion of silicon-oxygen bonds in peptide like ACE inhibitors to silicon fluoride bonds by using HF followed by the hydrolysis using sodium hydroxide to avoid any oligomer formation during direct hydrolysis. (J. Org. Chem. 2005, 70, 15, 5781-5789.)

Choudhry et al. reported in their poster abstract on a one-step F-18 labelling towards Si-¹⁸F derivatives (European Journal of Nuclear Medicine and Molecular Imaging Vol. 33, Supplement (EANM Athene2006), P394, p. 306, September 2006).
It turned out that diphenyl-tert-butyl-SiF derivatives (see scheme 1) are superior to triphenyl-Si-F, phenyl-dimethyl-Si-F and tert-butyl-dimethyl-SiF regarding hydrolytic stability. The title of the abstract suggests an instant labelling of biomolecules by this methods but examples are not presented or given elsewhere.

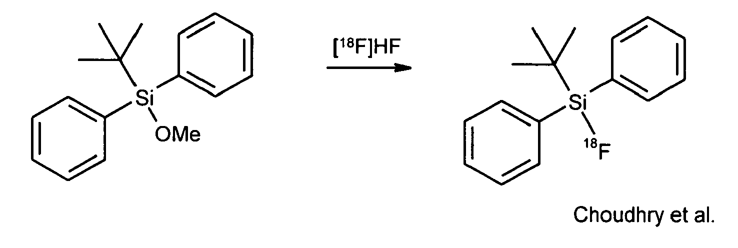

In the corresponding poster only methoxy was presented as leaving group regarding F-18 labelling of silicon-containing molecules. The disadvantage of methoxy is that the precursor (starting material) can not be separated easily from the F-18 labeled molecule or targeting agent: the retention time of both are too similar to achieve a convenient purification of the desired F-18 product. It can be shown that those leaving groups presented by alkoxy, comprising more than one carbon atom hydrogen, hydroxyl and aralkoxy are more suited to achieve a F-18 labeling and a subsequent successful separation of the desired F-18 labelled product from the precursor (compare HPLC chromatograms in scheme 2).

As used herein, the term "alkyl", by itself or as part of another group, refers to a straight chain or branched chain alkyl group with 1 to 20 carbon atoms such as, for example methyl, ethyl, propyl, isopropyl, butyl, isobutyl, tert-butyl, pentyl, isopentyl, neopentyl, heptyl, hexyl, decyl. Alkyl groups can also be substituted, such as by halogen atoms, hydroxyl groups, C1-C4-alkoxy groups or C6-C12-aryl groups (which, intern, can also be substituted, such as by 1 to 3 halogen atoms). "Alkenyl" and "alkinyl" are similarly defined, but contain at least one carbon-carbon double or triple bond, respectively.

The term "inorganic acid" and "organic acid" as employed herein refers to mineral acids, including but not limited to: acids such as carbonic, nitric, phosphoric, hydrochloric, perchloric or sulphuric acid or acidic salts such as potassium hydrogen sulphate or to appropriate organic acids which includes but not limited to: acids such as aliphatic, cycloaliphatic, aromatic, araliphatic, heterocyclic, carboxylic and sulphonic acids, examples of which are formic, acetic, trifluoracetic, propionic, succinic, glycolic, gluconic, lactic, malic, fumaric, pyruvic, benzoic, anthranilic, mesylic, fumaric, salicylic, phenylacetic, mandelic, embonic, methansulfonic, ethanesulfonic, benzenesulfonic, phantothenic, toluenesulfonic, trifluormethansulfonic and sulfanilic acid.

As used herein, the term "aryl", by itself or as part of another group, refers to monocyclic or bicyclic aromatic groups containing from 6 to 12 carbons in the ring portion, preferably 6-10 carbons in the ring portion, such as phenyl, naphthyl or tetrahydronaphthyl.

As used herein, the term "aralkyl", by itself or as part of another group, refers to an aryl as defined above substituted by an alkyl as defined above, whereas either the alkyl or the aryl (or both) may be attached to another group or molecule.

As used herein, the term "heteroaryl", by itself or as part of another group, refers to groups having 5 to 14 ring atoms; 6, 10 or 14 Π electrons shared in a cyclic array; and containing carbon atoms and 1, 2, 3 or 4 oxygen, nitrogen or sulfur heteroatoms Examples of heteroaryl groups are: thienyl, benzo[b]thienyl, naphtho[2,3-b]thienyl, thian-threnyl, furyl, pyranyl, isobenzofuranyl, benzoxazolyl, chromenyl, xanthenyl, phenoxythiinyl, 2H-pyrrolyl, pyrrolyl, imidazolyl, pyrazolyl, pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalazinyl, naphthyridinyl, quinazolinyl, cinnolinyl, pteridinyl, 4aH-carbazolyl, carbazolyl, carbolinyl, phenanthridinyl, acridinyl, perimidinyl, phenanthrolinyl, phenazinyl, isothiazolyl, phenothiazinyl, isoxazolyl, furazanyl und phenoxazinyl.

As used herein, the term "targeting agent" is directed to a compound or moiety that targets or directs the radionuclide attached to it to a specific site in a biological system. A targeting agent can be any compound or chemical entity that binds to or accumulates at a target site in a mammalian body, i.e. the compound localizes to a greater extent at the target site than to surrounding tissue. Targeting agents are also designated, in this specification, in particular in the structural formulae, as "biomolecules".

As used herein, the term "peptide" refers to a molecule comprising an amino acid sequence of at least two amino acids.

As used herein, the term "amino acid sequence" is defined herein as a polyamide obtainable by polycondensation of at least two amino acids.

As used herein, the term "amino acid" means any molecule comprising at least one amino group and at least one carboxyl group, but no peptide bond within the molecule. In other words, an amino acid is a molecule that has a carboxylic acid functionality and an amine nitrogen having at least one free hydrogen, preferably in alpha position thereto, but no amide bond in the molecule structure. Thus, a dipeptide having a free amino group at the N-terminus and a free carboxyl group at the C-terminus is not to be considered as a single "amino acid" within the above definition.

An amide bond as used herein means any covalent bond having the structure 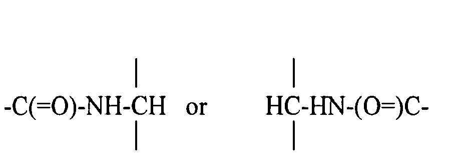 wherein the carbonyl group is provided by one molecule and the NH-group is provided by the other molecule to be joined. The amide bonds between two adjacent amino acid residues which are obtained from such a polycondensation are defined as "peptide bonds". Optionally, the nitrogen atoms of the polyamide backbone (indicated as NH above) may be independently alkylated, e.g. with -C₁-C₆-alkyl, preferably -CH₃.

For the purpose of the specification, an amino acid residue is derived from the corresponding amino acid by forming a peptide bond with another amino acid.

For the purpose of the specification, an amino acid sequence may comprise naturally occurring and/or synthetic amino acid residues, proteinogenic and/or non-proteinogenic amino acid residues. The non-proteinogenic amino acid residues may be further classified as (a) homoanalogues of proteinogenic amino acids, (b) β-homoanalogues of proteinogenic amino acid residues and (c) further non-proteinogenic amino acid residues.

Accordingly, the amino acid residues are derived from the corresponding amino acids, e.g. from
proteinogenic amino acids, namely Ala, Arg, Asn, Asp, Cys, Gln, Glu, Gly, His, Ile, Leu, Lys, Met, Phe, Pro, Ser, Thr, Trp, Tyr and Val; or
non-proteinogenic amino acids, such as
homoanalogues of proteinogenic amino acids wherein the sidechain has been extended by a methylene group, e.g. homoalanine (Hal), homoarginine (Har), homocysteine (Hcy), homoglutamine (Hgl), homohistidine (Hhi), homoisoleucine (Hil), homoleucine (Hle), homolysine (Hly), homomethionine (Hme), homophenylalanine (Hph), homoproline (Hpr), homoserine (Hse), homothreonine (Hth), homotryptophane (Htr), homotyrosine (Hty) and homovaline (Hva);
β -homoanalogues of proteinogenic amino acids wherein a methylene group has been inserted between the α-carbon and the carboxyl group yielding β-amino acids, e.g. β-homoalanine (βHal), β-homoarginine (βHar), β-homoasparagine (βHas), β-homocysteine (βHcy), β-homoglutamine (βHgl), β-homohistidine (βHhi), β-homoisoleucine (βHil), β-homoleucine (βHle), β-homolysine (βHly), β-homomethionine (βHme), β-homophenylalanine (βHph), β-homoproline (βHpr), β-homoserine (βHse), β-homothreonine (βHth), β-homotryptophane (βHtr), β-homotyrosine (βHty) and β-homovaline (βHva);
further non-proteinogenic amino acids, e.g. α-aminoadipic acid (Aad), β-aminoadipic acid (βAad), α-aminobutyric acid (Abu), α-aminoisobutyric acid (Aib), β-alanine (βAla), 4-aminobutyric acid (4-Abu), 5-aminovaleric acid (5-Ava), 6-aminohexanoic acid (6-Ahx), 8-aminooctanoic acid (8-Aoc), 9-aminononanoic acid (9-Anc), 10-aminodecanoic acid (10-Adc), 12-aminododecanoic acid (12-Ado), α-aminosuberic acid (Asu), azetidine-2-carboxylic acid (Aze), β-cyclohexylalanine (Cha), citrulline (Cit), dehydroalanine (Dha), γ-carboxyglutamic acid (Gla), α-cyclohexylglycine (Chg), propargylglycine (Pra), pyroglutamic acid (Glp), α-tert-butylglycine (Tle), 4-benzoylphenylalanine (Bpa), δ-hydroxylysine (Hyl), 4-hydroxyproline (Hyp), alloisoleucine (aIle), lanthionine (Lan), (1-naphthyl)alanine (1-Nal), (2-naphthyl)alanine (2-Nal), norleucine (Nle), norvaline (Nva), ornithine (Orn), phenylglycin (Phg), pipecolic acid (Pip), sarcosine (Sar), selenocysteine (Sec), statine (Sta), β-thienylalanine (Thi), 1,2,3,4-tetrahydroisochinoline-3-carboxylic acid (Tic), allo-threonine (aThr), thiazolidine-4-carboxylic acid (Thz), γ-aminobutyric acid (GABA), iso-cysteine (iso-Cys), diaminopropionic acid (Dpr), 2,4-diaminobutyric acid (Dab), 3,4-diaminobutyric acid (γ,βDab), biphenylalanine (Bip), phenylalanine substituted in para-position with -C₁-C₆-alkyl, -halide, -NH₂ or -CO₂H (Phe(4-R) wherein R = -C₁-C₆-alkyl, -halide, -NH₂, or - CO₂H); peptide nucleic acids (PNA, cf. P.E. Nielsen, Acc.Chem.Res. 32, 624-30)
or their N-alkylated analogues, such as their N-methylated analogues.

Cyclic amino acids may be proteinogenic or non-proteinogenic, such as Pro, Aze, Glp, Hyp, Pip, Tic and Thz.

For further examples and details reference can be made to e.g. J.H. Jones, J. Peptide Sci. 2003, 9, 1-8.

The terms "non-proteinogenic amino acid" and "non-proteinogenic amino acid residue" also encompasses derivatives of proteinogenic amino acids. For example, the sidechain of a proteinogenic amino acid residue may be derivatized thereby rendering the proteinogenic amino acid residue "non-proteinogenic". The same applies to derivatives of the C-terminus and/or the N-terminus of a proteinogenic amino acid residue terminating the amino acid sequence.

For the purpose of the specification, a proteinogenic amino acid residue is derived from a proteinogenic amino acid selected from the group consisting of Ala, Arg, Asn, Asp, Cys, Gln, Glu, Gly, His, Ile, Leu, Lys, Met, Phe, Pro, Ser, Thr, Trp, Tyr and Val either in L- or D-configuration; the second chiral center in Thr and Ile may have either R- or S-configuration. Therefore, for example, any posttranslational modification of an amino acid sequence, such as N-alkylation, which might naturally occur renders the corresponding modified amino acid residue "non-proteinogenic", although in nature said amino acid residue is incorporated in a protein.

Examples for peptides which can be used as targeting agents in the compounds of the present invention are, but are not limited to, somatostatin and derivatives thereof and related peptides, neuropeptide Y and derivatives thereof and related peptides, bombesin and derivatives thereof and related peptides, gastrin, gastrin releasing peptide derivatives thereof and related peptides, epidermal growth factor (EGF of various origin), insulin growth factor (IGF) and IGF-1, LHRH agonists and antagonists, transforming growth factors, particularly TGF-α, angiotensin, cholecystokinin (CCK) and analogs, neurotensin and analogs, thyrotropin releasing hormone, prolactin, tumor necrosis factor, IL-1, IL-2, IL-4 or IL-6, interferons, VIP and related peptides, PACAP and related peptides. Such peptides comprise from 4 to 100 amino acids, wherein the amino acids are selected from natural and non-natural amino acids and also comprise modified natural and non-natural amino acids.

In other preferred embodiments, the targeting agent is selected to be an oligonucleotide.

For the purposes of this invention, the term "oligonucleotide" shall have the following meaning: short sequences of nucleotides, typically with twenty or fewer bases. Examples are, but are not limited to, molecules named and cited in: "The aptamers handbook. Functional oligonuclides and their application" by Svenn Klussmann, Wiley-VCH, 2006. An example for such an oligonucleotide is TTA1 *(*J. Nucl. Med., 2006, April, 47(4), 668-78).

For the purpose of this invention, the term "aptamer" refers to an oligonucleotide, comprising from 4 to 100 nucleotides, wherein at least two single nucleotides are connected to each other via a phosphodiester linkage. Said aptamers have the ability to bind specifically to a target molecule (see e.g. M Famulok, G Mayer, Aptamers as Tools in Molecular Biology and Immunology, In: Combinatorial Chemistry in Biology, Current Topics in Microbiology and Immunology (M Famulok, CH Wong, EL Winnacker, Eds.), Springer Verlag Heidelberg, 1999, Vol. 243, 123-136). There are many ways known to the skilled person of how to generate such aptamers that have specificity for a certain target molecule. An example is given in WO 01/09390, the disclosure of which is hereby incorporated by reference. Said aptamers may comprise substituted or non-substituted natural and non-natural nucleotides. Aptamers can be synthesized in vitro using e.g. an automated synthesizer. Aptamers according to the present invention can be stabilized against nuclease degradation e.g. by the substitution of the 2'-OH group versus a 2'-fluoro substituent of the ribose backbone of pyrimidine and versus 2'-O-methyl substituents in the purine nucleic acids. In addition, the 3' end of an aptamer can be protected against exonuclease degradation by inverting the 3' nucleotide to form a new 5'-OH group, with a 3' to 3' linkage to a penultimate base.

For the purpose of this invention, the term "nucleotide" refers to molecules comprising a nitrogen-containing base, a 5-carbon sugar, and one or more phosphate groups. Examples of said base comprise, but are not limited to, adenine, guanine, cytosine, uracil, and thymine. Also non-natural, substituted or non-substituted bases are included. Examples of 5-carbon sugar comprise, but are not limited to, D-ribose, and D-2-desoxyribose. Also other natural and non-natural, substituted or non-substituted 5-carbon sugars are included. Nucleotides as used in this invention may comprise from one to three phosphates.

Small molecules effective for targeting certain sites in a biological system can also be used as the targeting agent.

For the purposes of this invention, the term "small molecule" shall have the following meaning: a small molecule is a compound that has a molecular mass of 200 to 800 and that contains a primary and/or secondary amine to which compounds of Formula I and II are coupled via L. Such targeting moieties are known in the art, so are methods for preparing them.

As used herein, the term "small molecule" is a compound that has a molecular mass of 200 to 800 and that contains a primary and/or secondary amine to which the novel compounds as defined herein are coupled via the linker L. Such targeting agents are known in the art, so are methods for preparing them.

Preferred as targeting agents are sugars, oligosaccharides, polysaccharides, aminoacids, nucleic acids, nucleotides, nucleosides, oligonucleotides, proteins, peptides, peptidomimetics, antibodies, aptamers, lipids, hormones (steroid and nonsteroid), neurotransmitters, drugs (synthetic or natural), dendrimers, fullerenes, virus particles and other targeting molecules (e.g. cancer targeting molecules).

As used herein the term "pharmaceutically acceptable salt" comprises salts of inorganic and organic acids such an mineral acids, including, but not limited to, acids such as carbonic, nitric or sulfuric acid, or organic acids, including, but not limited to acids such as aliphatic, cycloaliphatic, aromatic, araliphatic, heterocyclic, carboxylic and sulphonic acids, examples of which are formic, acetic, trifluoroacetic, propionic, succinic, glycolic, gluconic, lactic, malic, fumaric, pyruvic, benzoic, anthranilic, mesylic, salicylic, phenylacetic, mandelic, embonic, methansulfonic, ethanesulfonic, benzenesulfonic, phantothenic, toluenesulfonic and sulfanilic acid.

If a chiral center or another form of an isomeric center is present in a compound according to Formula I, II or III of the present invention, all forms of such isomers, including enantiomers and diastereoisomers, are intended to be covered herein. Compounds containing a chiral center may be used as a racemic mixture or as an enantiomerically enriched mixture, or the racemic mixture may be separated using well-known techniques and an individual enantiomer maybe used alone. In cases in which compounds have unsaturated carbon-carbon double bonds, both the cis-isomer and trans-isomers are within the scope of this invention. In cases in which compounds may exist in tautomeric forms, such as keto-enol tautomers, each tautomeric form is contemplated as being included within this invention whether existing in equilibrium or predominantly in one form.

### Detailed Description of Invention

Examples of syntheses of compounds of formula I and II are shown below. Model fluoro silanes using non-radioactive fluoride (¹⁹F) were synthesized in order to test the procedures and used to confirm the preparation of the corresponding labelled derivatives.

### I. Synthesis of precursors (silanes) via nucleophilic substitution

The following scheme describes the general synthetic route of suitable silyl building blocks which can be coupled to biomolecules followed by fluorination or subsequent direct radiolabelling towards the corresponding ¹⁸F labelled compounds of formula I. Compound 7 was prepared by nucleophilic substitution of silyl chlorides with aryl metal complexes followed by acidic deprotection (4) and Jones oxidation (5) to generate functionalities like carbonic acids 5 or active esters 6 for coupling to biomolecules (7). The coupling can be performed using well known coupling reagents like EDCI or DCC. Silane 7 was then fluorinated using pottasium fluoride and acetic acid to yield the tracer 8. It turned out that the use of acid guarantees improved yields for this reaction and also for the reactions described in following sections II, III, IV and V. This result is surprising due to the fact that the expected or potential formation of hydrogen fluoride would rather stop the reaction than accelerating it.

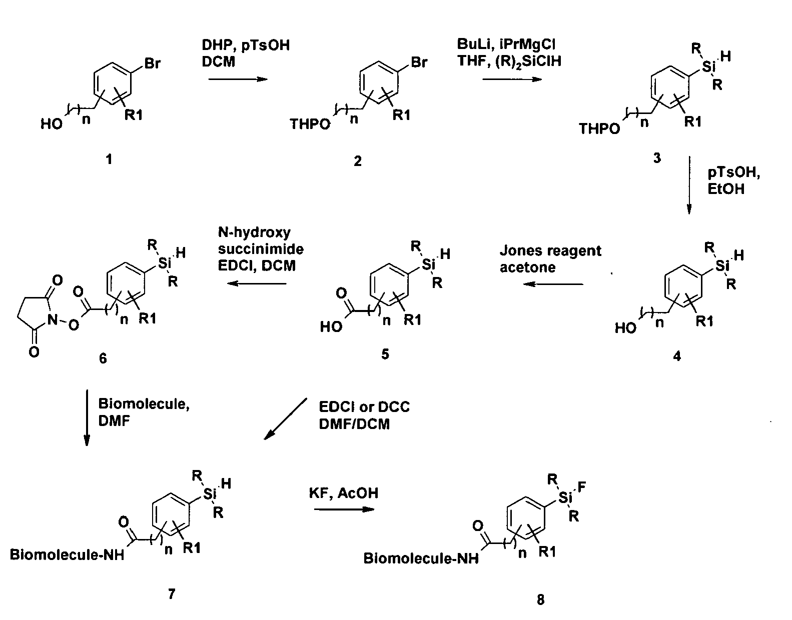

### II. Synthesis of precursors (silanoles) via nucleophilic substitution

Alternatively, silanoles were applied as labelling precursors. Here silane 6 was subjected to an oxidation to silanole 9 prior coupling to the biomolecule to yield precursors of type 10 which were readily fluorinated with pottasium fluoride, potassium carbonate, kryptofix and acetic acid to give tracers of type 8.

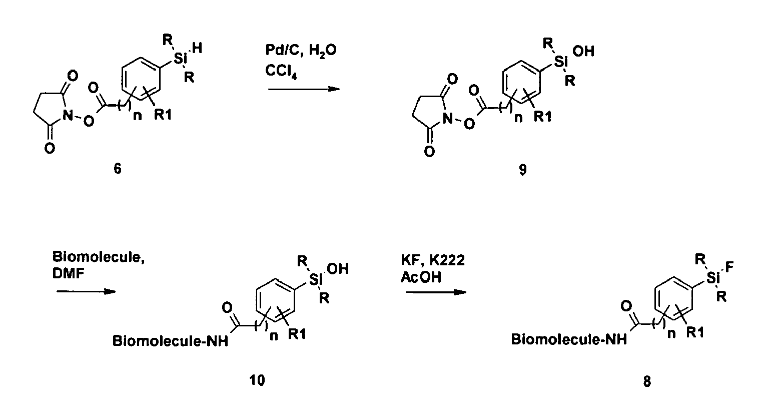

### III. Synthesis of precursors via carbene insertion

Benzyl diazoacetate 12 was prepared by diazotization of benzyl glycine by the method described by N. E. Searle (Org. Synth., Coll. Vol. 4, p. 424, 1963; Vol. 36, p. 25, 1956) for the synthesis of ethyl diazoacetate. Addition of benzyl diazoacetate 12 to a mixture of di-alkyl chlorosilane and a catalytic amount of Rh₂(OAc)₄ in anhydrous dichloro-methane yields a chlorosilane intermediate which can either be added to a mixture of alcohol, imidazole and 4-DMAP in dry DMF to give alkoxysilanes 13. Or, respectively, the chlorosilane intermediate was treated with NEt₃ and H₂O to give the silanol 13 (R¹=OH). The benzyl ester group of the silanol 13 can then be cleaved off by hydrogenation in presence of 10% Pd/C catalyst to give 14 which can be coupled to biomolecules 15 followed by fluorination with potassium fluoride, potassium carbonate, kryptofix, and acetic acid to 16.

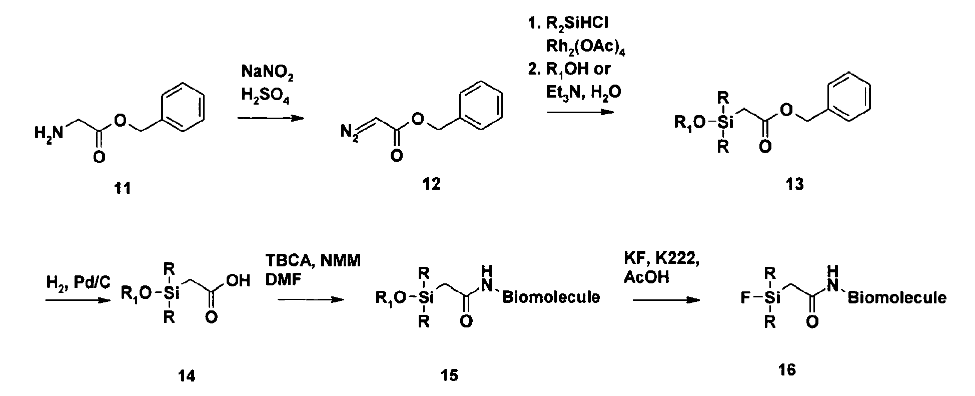

### IV. Synthesis of precursors and fluorinated targeting agents starting from commercially available Si-derivatives:

The commercially available diisopropyl silicon amine 17 reflects an example of silicon derivatives which can be used as described in the following scheme in the synthesis of a precursors 19 that can be coupled to a targeting agent towards 20 followed by fluorination with potassium fluoride, potassium carbonate, kryptofix, and acetic acid to 21.

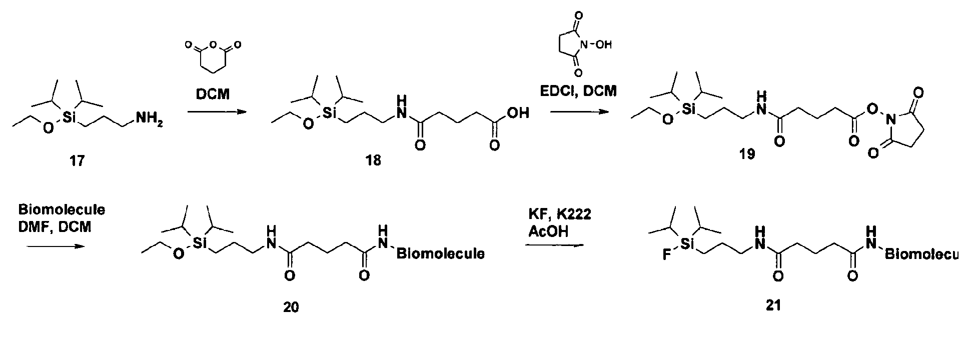

### V. Synthesis of precursors via hydrosilylation

Suitable silicon building blocks for labelling can also be prepared via hydrosilylation as shown in the following scheme. The functionalized alkene 26 can be transformed to chlorosilane 27 using the Karstedt Catalyst Pt₂{[(CH₂=CH)Me₂Si]₂O}₃. Treatment with alcohols or water yield silanols or alkoxysilanes 28 which can be coupled to biomolecules to receive labeling precursors of type 29 applicable for subsequent fluorination with potassium fluoride, potassium carbonate, kryptofix and acetic acid to 30.

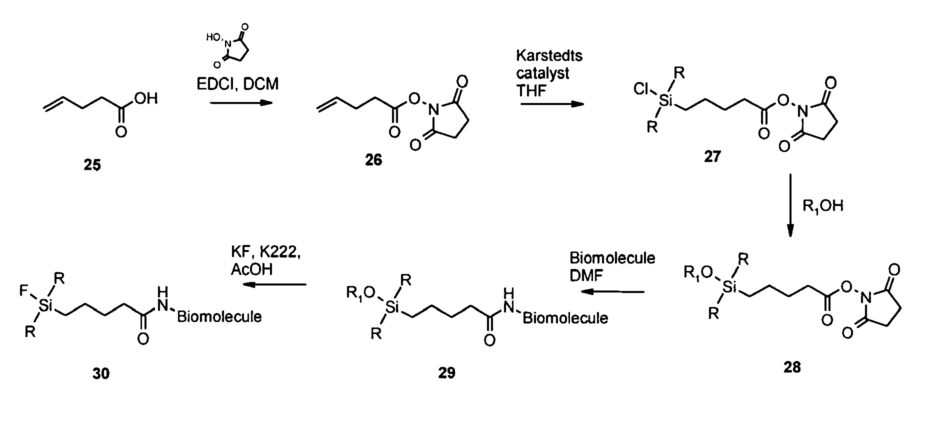

### Experimental Part

### EXAMPLE I

### PART A

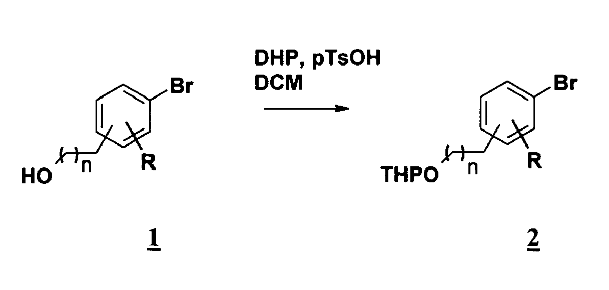

### General Method

To a solution of 53.5 mmol of **1** in 220 mL dichloro-methane 36 mL 3,4-dihydro-2H-pyran and 202 mg pyridinium toluene-4-sulfonate was added. After the reaction mixture was stirred for 16 hours at 23°C it was added to a solution of sodium bicarbonate. The organic extract was washed with brine and dried over sodium sulfate. After filtration and removal of the solvent the crude product was purified by chromatography on silica gel to give **2** in 77-98% yield.
(RS)-2-(4-Bromo-benzyloxy)-tetrahydro-pyran 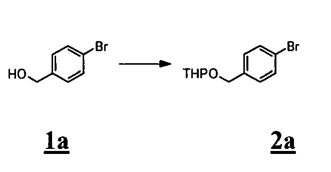 ¹H-NMR (CDCI₃): δ = 7.47 (2H), 7.24 (2H), 4.73 (1H), 4.69 (1H), 4.46 (1H), 3.89 (1H), 3.54 (1H), 1.86 (1H), 1.74 (1H), 1.69-1.50 (4H) ppm.
(RS)-2-[2-(4-Bromo-phenyl)-ethoxy]-tetrahydro-pyran 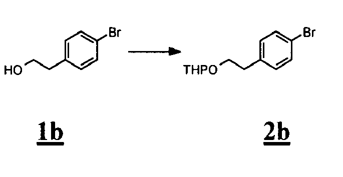 ¹H-NMR (CDCl₃): δ = 7.51 (2H), 7.29 (2H), 5.20-4.50 (2H), 3.65 (2H), 1.20 (3H), 1.04 (6H), 0.96 (6H) ppm.
(RS)-2-[3-(4-Bromo-phenyl)-propoxy]-tetrahydro-pyran 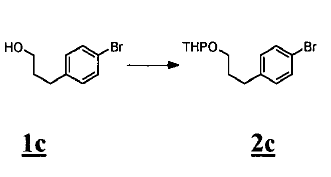 ¹H-NMR (CDCl₃): δ = 7.39 (2H), 7.07 (2H), 4.56 (1H), 3.86 (1H), 3.75 (1H), 3.50 (1H), 3.39 (1H), 2.67 (1H), 1.94-1.48 (9H) ppm.
(RS)-2-[2-(3-Bromo-phenyl)-ethoxy]-tetrahydro-pyran 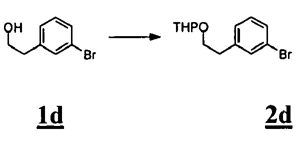 ¹H-NMR (CDCl₃): δ = 7.41 (1H), 7.34 (1H), 7.19-7.11 (2H), 4.59 (1H), 3.93 (1H), 3.72 (1H), 3.60 (1H), 3.45 (1H), 2.88 (2H), 1.87-1.44 (6H) ppm.
(RS)-2-[3-(3-Bromo-phenyl)-propoxy]-tetrahydro-pyran 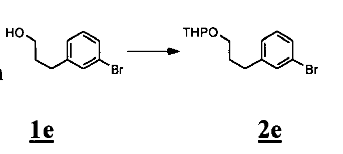 ¹H-NMR (CDCl₃): δ = 7.36 (1H), 7.31 (1H), 7.17-7.11 (2H), 4.57 (1H), 3.86 (1H), 3.76 (1H), 3.50 (1H), 3.39 (1H), 2.68 (2H), 1.91 (2H), 1.86-1.49 (6H) ppm.

### PART B

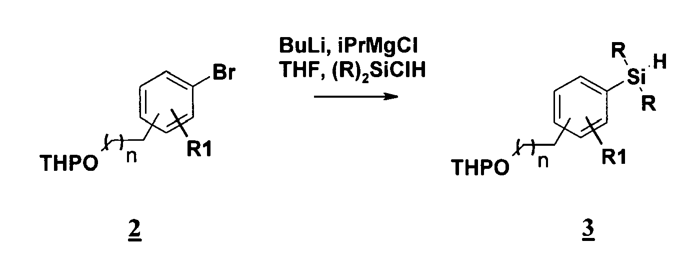

### General Method

14.2 mL of a 2M isopropylmagnesium bromide solution in tetrahydrofuran (THF) was diluted with 140 mL THF and cooled to 5°C. 22.6 mL of a 2.5M solution of butyllithium in n-hexane was added followed by the solution of 14.2 mmol **2** in 12 mL THF. After 2 hours at 5-10°C 17.6 g chloro-diisopropyl-silane was added, the cooling bath removed and stirring continued for 2 hours. The reaction mixture was added to a solution of sodium bicarbonate and extracted with ethyl acetate. The combined organic extracts were washed with brine and dried over sodium sulfate. After filtration and removal of the solvent the crude product was purified by chromatography on silica gel to give 93-99% of **3**.
(RS)-Diisopropyl-[4-(tetrahydro-pyran-2-yloxymethyl)-phenyl]-silane 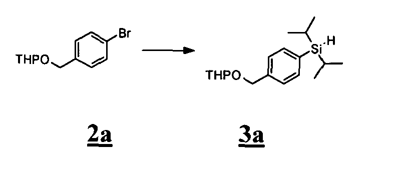 ¹H-NMR (CDCI₃): δ = 7.43 (2H), 7.28 (2H), 4.73 (1H), 4.66 (1H), 4.42 (1H), 3.86 (2H), 3.49 (1H), 1.89-1.43 (6H), 1.14 (2H), 0.99 (6H), 0.92 (6H) ppm.
(RS)-Diisopropyl-{4-[2-(tetrahydro-pyran-2-yloxy)-ethyl]-phenyl}-silane 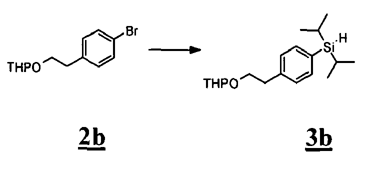 ¹H-NMR (CDCI₃): δ = 7.43 (2H), 7.22 (2H), 4.60 (1H), 3.98-3.87 (2H), 3.71-3.60 (2H), 3.42 (1H), 2.91 (2H), 1.79 (1H), 1.69 (1H), 1.63-1.43 (4H), 1.21 (2H), 1.05 (6H), 0.97 (6H) ppm.
(RS)-Diisopropyl-{4-[3-(tetrahydro-pyran-2-yloxy)-propyl]-phenyl}-silane 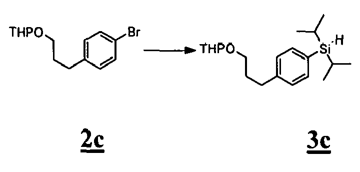 ¹H-NMR (CDCl₃): δ = 7.42 (2H), 7.19 (2H), 4.59 (1H), 3.92 (1H), 3.87 (1H), 3.78 (1H), 3.50 (1H), 3.42 (1H), 2.71 (2H), 1.94 (2H), 1.84 (1H), 1.72 (1H), 1.63-1.49 (4H), 1.22 (2H), 1.06 (6H), 0.99 (6H) ppm.
(RS)-Diisopropyl-{3-[2-(tetrahydro-pyran-2-yloxy)-ethyl]-phenyl}-silane 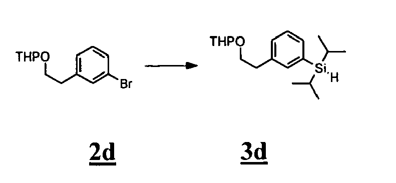 ¹H-NMR (CDCl₃): δ = 7.39 (1H), 7.34 (1H), 7.29-7.24 (2H), 4.60 (1H), 3.95 (1H), 3.92 (1H), 3.72 (1H), 3.62 (1H), 3.44 (1H), 2.91 (2H), 1.80 (1H), 1.68 (1H), 1.62-1.43 (4H), 1.22 (2H), 1.06 (6H), 0.99 (6H) ppm.
(RS)-Diisopropyl-{3-[3-(tetrahydro-pyran-2-yloxy)-propyl]-phenyl}-silane 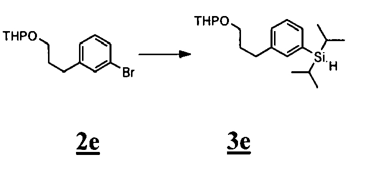 ¹H-NMR (CDCl₃): δ = 7.83 (2H), 7.26 (1H), 7.21 (1H), 4.58 (1H), 3.92 (1H), 3.88 (1H), 3.78 (1H), 3.50 (1H), 3.40 (1H), 2.70 (2H), 1.93 (2H), 1.85 (1H), 1.73 (1H), 1.64-1.59 (4H), 1.23 (2H), 1.06 (6H), 0.99 (6H) ppm.
(RS)-Diphenyl-{4-[2-(tetrahydro-pyran-2-yloxy)-ethyl]-phenyl}-silane 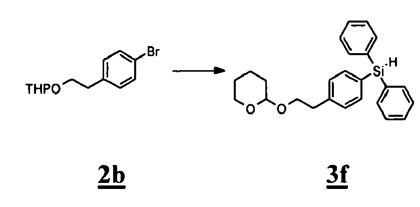 ¹H-NMR (CDCI₃): δ = 7.62 (4H), 7.55 (2H), 7.50-7.37 (6H), 7.31 (2H), 5.50 (1H), 4.64 (1H), 3.99 (1H), 3.77 (1H), 3.67 (1H), 3.49 (1H), 2.97 (2H), 1.90-1.48 (6H) ppm.
(RS)-Diisobutyl-[4-(tetrahydro-pyran-2-yloxymethyl)-phenyl]-silane 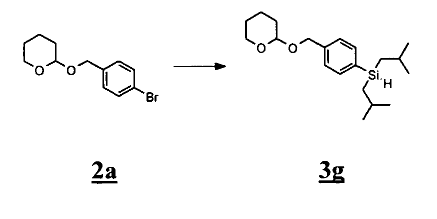 ¹H-NMR (CDCl₃): δ = 7.52 (2H), 7.35 (2H), 4.80 (1H), 4.73 (1H), 4.49 (1H), 4.38 (1H), 3.93 (1H), 3.56 (1H), 1.95-1.49 (8H), 0.94 (6H), 0.92 (6H), 0.82 (4H) ppm.
(RS)-Diisobutyl-{4-[2-(tetrahydro-pyran-2-yloxy)-ethyl]-phenyl}-silane 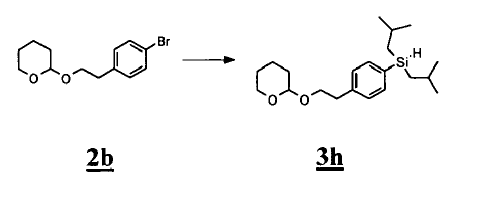 ¹H-NMR (CDCI₃): δ = 7.45 (2H), 7.23 (2H), 4.60 (1H), 4.36 (1H), 3.95 (1H), 3.71 (1H), 3.63 (1H), 3.43 (1H), 2.91 (2H), 1.87-1.43 (8H), 0.93 (6H), 0.91 (6H), 0.81 (4H) ppm.

### PART C

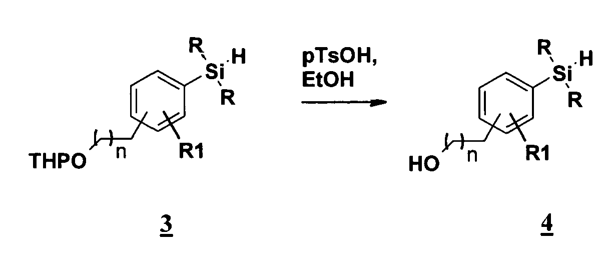

### General Method

To a solution 9.36 mmol **3** in 120 mL ethanol were added 1.61 g p-toluenesulfonic acid monohydrate and the mixture was stirred for 2 hours at 23°C. The reaction mixture was added to a solution of sodium bicarbonate extracted with dichloromethane. The combined organic extracts were washed with brine and dried over sodium sulfate. After filtration and removal of the solvent the crude product was purified by chromatography on silica gel to give 70-93% of **4.**
(4-Diisopropylsilanyl-phenyl)-methanol 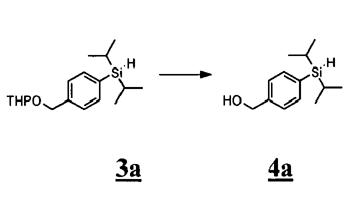 ¹H-NMR (CDCl₃): δ = 7.51 (2H), 7.35 (2H), 4.70 (2H), 3.94 (1H), 1.64 (1H), 1.23 (2H), 1.06 (6H), 0.98 (6H) ppm.
2-(4-Diisopropylsilanyl-phenyl)-ethanol 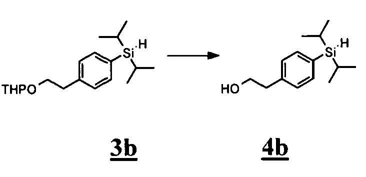 ¹H-NMR (CDCl₃): δ = 7.46 (2H), 7.22 (2H), 3.93 (1H), 3.88 (2H), 2.87 (2H), 1.53 (1H), 1.22 (2H), 1.06 (6H), 0.99 (6H) ppm.
3-(4-Diisopropylsilanyl-phenyl)-propan-1-ol 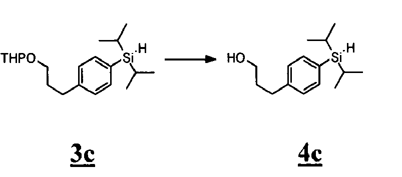 ¹H-NMR (CDCl₃): δ = 7.44 (2H), 7.19 (2H), 3.92 (1H), 3.69 (2H), 2.71 (2H), 1.91 (2H), 1.30 (1H), 1.21 (2H), 1.06 (6H), 0.99 (6H) ppm.
3-(3-Diisopropylsilanyl-phenyl)-propan-1-ol 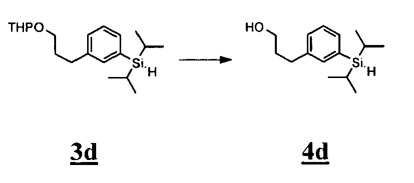 ¹H-NMR (CDCl₃): δ = 7.33 (2H), 7.26 (1H), 7.21 (1H), 3.92 (1H), 3.69 (2H), 2.71 (2H), 1.90 (2H), 1.30 (1H), 1.22 (2H), 1.06 (6H), 0.99 (6H) ppm.
2-(3-Diisopropylsilanyl-phenyl)-ethanol 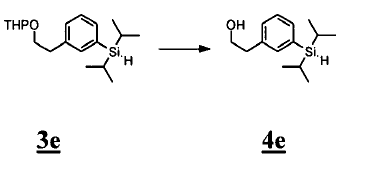 ¹H-NMR (CDCl₃): δ = 7.39 (1H), 7.37 (1H), 7.30 (1H), 7.24 (1H), 3.93 (1H), 3.87 (2H), 2.87 (2H), 1.39 (1H), 1.23 (2H), 1.06 (6H), 0.99 (6H) ppm.
(4-Diisobutylsilanyl-phenyl)-methanol 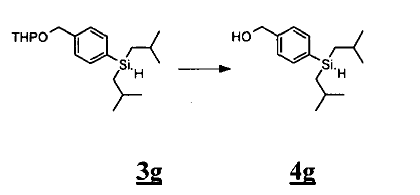 ¹H-NMR (CDCl₃): δ = 7.59 (2H), 7.39 (2H), 4.74 (2H), 4.43 (1H), 1.79 (2H), 1.68 (1H), 0.99 (6H), 0.97 (6H), 0.87 (4H) ppm. 2-(4-Diisobutylsilanyl-phenyl)-ethanol 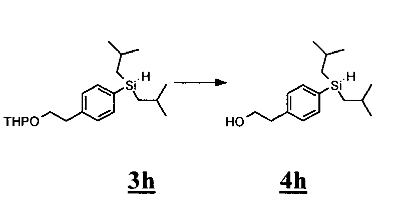 ¹H-NMR (CDCl₃): δ = 7.53 (2H), 7.26 (2H), 4.42 (1H), 3.92 (2H), 2.92 (2H), 1.80 (2H), 1.51 (1H), 0.97 (6H), 0.99 (6H), 0.87 (4H) ppm.

### PART D

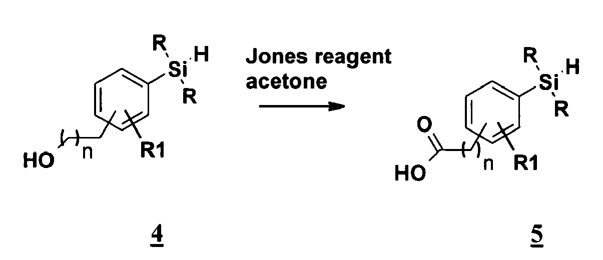

### General Method

To a solution of 2 mmol **4** in 14 mL acetone was added at 0°C 2.25 mL of Jones reagent. After 15 minutes water was added and the mixture extracted with ethyl acetate. The combined organic extracts were washed with brine and dried over sodium sulfate. After filtration and removal of the solvent the crude product was purified by chromatography on silica gel to give 52-70% of **5**. 4-Diisopropylsilanyl-benzoic acid 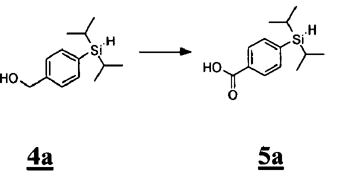 ¹H-NMR (CDCI₃): δ = 8.07 (2H), 7.64 (2H), 3.99 (1H), 1.27 (2H), 1.08 (6H), 0.99 (6H) ppm.
(4-Diisopropylsilanyl-phenyl)-acetic acid 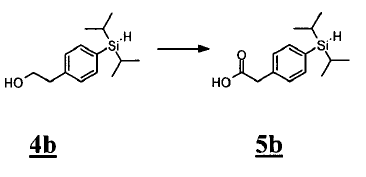 ¹H-NMR (CDCl₃): δ = 7.48 (2H), 7.28 (2H), 3.93 (1H), 3.65 (2H), 1.22 (2H), 1.06 (6H), 0.99 (6H) ppm. 3-(4-Diisopropylsilanyl-phenyl)-propionic acid 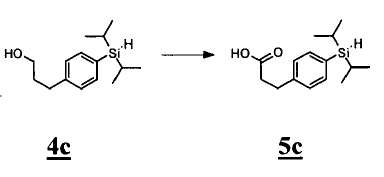 ¹H-NMR (CDCl₃): δ = 7.45 (2H), 7.20 (2H), 3.93 (1H), 2.97 (2H), 2.70 (2H), 1.22 (2H), 1.06 (6H), 0.99 (6H) ppm.
3-(3-Diisopropylsilanyl-phenyl)-propionic acid 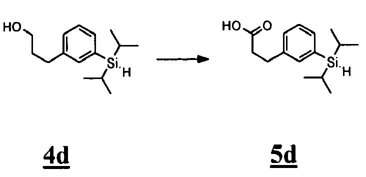 ¹H-NMR (CDCI₃): δ = 7.37 (1H), 7.35 (1H), 7.28 (1H), 7.22 (1H), 3.92 (1H), 2.96 (2H), 2.69 (2H), 1.22 (2H), 1.06 (6H), 0.98 (6H) ppm.
(3-Diisopropylsilanyl-phenyl)-acetic acid 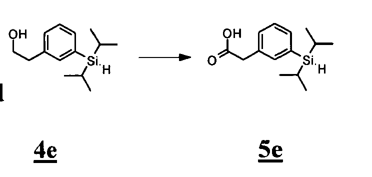 ¹H-NMR (CDCl₃): δ = 7.45-7.39 (2H), 7.34-7.30 (2H), 3.93 (1H), 3.65 (2H), 1.22 (2H), 1.06 (6H), 0.99 (6H) ppm. 4-Diisobutylsilanyl-benzoic acid 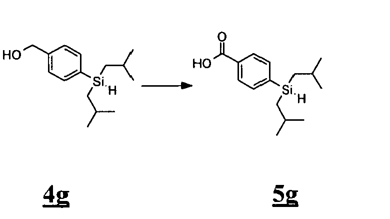 ¹H-NMR (CDCl₃): δ = 8.07 (2H), 7.67 (2H), 4.43 (1H), 1.75 (2H), 0.94 (6H), 0.93 (6H), 0.86 (4H) ppm.
(4-Diisobutylsilanyl-phenyl)-acetic acid 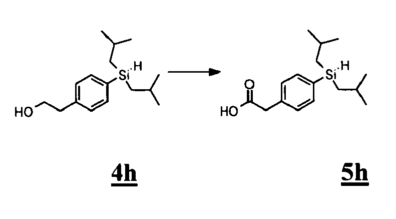 ¹H-NMR (CDCl₃): δ = 7.50 (2H), 7.27 (2H), 4.37 (1H), 3.65 (2H), 1.74 (2H), 0.94 (6H), 0.93 (6H), 0.82 (4H) ppm.

### PART E

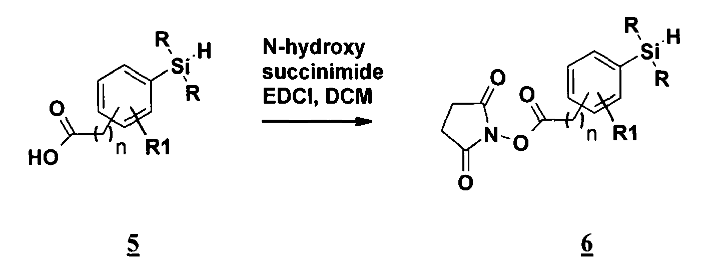

### General Method

To a solution of 600 µmol **5** in 6 mL dichloro-methane were added 76 mg N-hydroxy-succinimide, 126 mg (3-Dimethylamino-propyl)-ethyl-carbodiimide hydrochloride and the mixture was stirred for 16 hours at 23°C. After addition of water and extraction with dichloro-methane the combined organic extracts were dried over sodium sulfate. After filtration and solvent evaporation the crude product was purified by chromatography on silica gel to give 73-99% of **6**.
4-Diisopropylsilanyl-benzoic acid 2,5-dioxo-pyrrolidin-1-yl ester 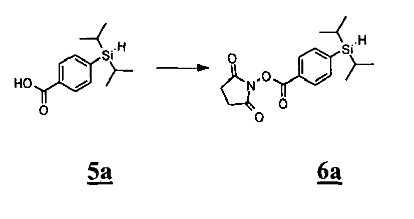 ¹H-NMR (CDCl₃): δ = 8.09 (2H), 7.67 (2H), 3.99 (1H), 2.91 (4H), 1.27 (2H), 1.07 (6H), 0.98 (6H) ppm.
(4-Diisopropylsilanyl-phenyl)-acetic acid 2,5-dioxo-pyrrolidin-1-yl ester 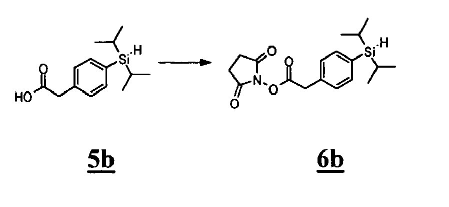 ¹H-NMR (CDCl₃): δ = 7.51 (2H), 7.32 (2H), 3.93 (3H), 2.83 (4H), 1.22 (2H), 1.06 (6H), 0.98 (6H) ppm.
3-(4-Diisopropylsilanyl-phenyl)-propionic acid 2,5-dioxo-pyrrolidin-1-yl ester 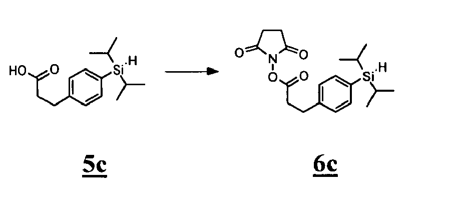 ¹H-NMR (CDCl₃): δ = 7.46 (2H), 7.21 (2H), 3.92 (1H), 3,06 (2H), 2.93 (2H), 2.85
(4H), 1.21 (2H), 1.06 (6H), 0.98 (6H) ppm. ¹H-NMR (CDCI₃): δ = 7.46 (2H), 7.35 (2H), 3.94 (3H), 2.83 (4H), 1.22 (2H), 1.06
(6H), 0.98 (6H) ppm.
3-(3-Diisopropylsilanyl-phenyl)-propionic acid 2,5-dioxo-pyrrolidin-1-yl ester 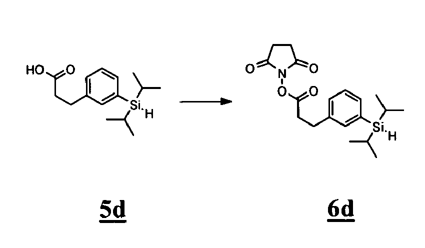 ¹H-NMR (CDCl₃): δ = 7.38 (1H), 7.35 (1H), 7.30 (1H), 7.23 (1H), 3.92 (1H), 3.05 (2H), 2.92 (2H), 2.85 (4H), 1.22 (2H), 1.06 (6H), 0.98 (6H) ppm.
(3-Diisopropylsilanyl-phenyl)-acetic acid 2,5-dioxo-pyrrolidin-1-yl ester 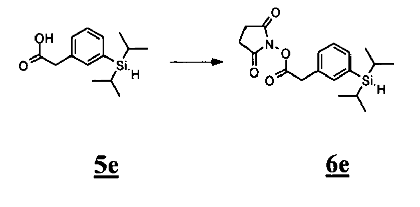 4-Diisobutylsilanyl-benzoic acid 2,5-dioxo-pyrrolidin-1-yl ester 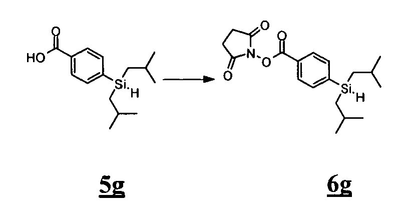 ¹H-NMR (CDCl₃): δ = 8.08 (2H), 7.69 (2H), 4.41 (1H), 2.91 (4H), 1.73 (2H), 0.93 (6H), 0.91 (6H), 0.86 (4H) ppm.
(4-Diisobutylsilanyl-phenyl)-acetic acid 2,5-dioxo-pyrrolidin-1-yl ester 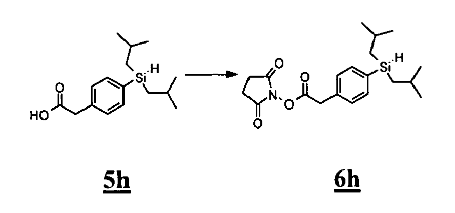 ¹H-NMR (CDCl₃): δ = 7.53 (2H), 7.32 (2H), 4.38 (1H), 3.93 (2H), 2.83 (4H), 1.74 (2H), 0.93 (6H), 0.92 (6H), 0.82 (4H) ppm.

### PART F1

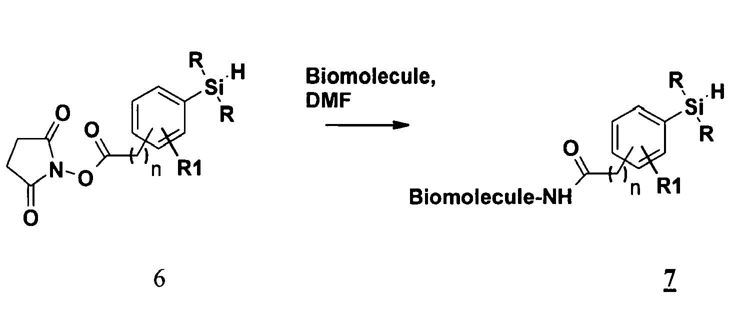

### General Method

0.05 mmol biomolecule or resin bound protected peptide suspended in 2 ml DMF was treated with 0.15 mmol silane active ester 6 for 12h. The reaction mixture was filtered and in case of the resin bound product, the resin was washed with DMF and dichloro-methane. After cleavage from the resin with 1 ml of a mixture of 85% TFA, 5% water, 5% phenol, and 5% triisopropylsilane, the product was precipitated with MTBE and purified by HPLC to give 12% of 7. Reaction mixtures with soluble biomolecules were evaporated and the residue was purified by column chromatography.
4-(Diisopropylsilanyl)-phenyl)-acetyl-Val-ßAla-Phe-Gly-NH₂ 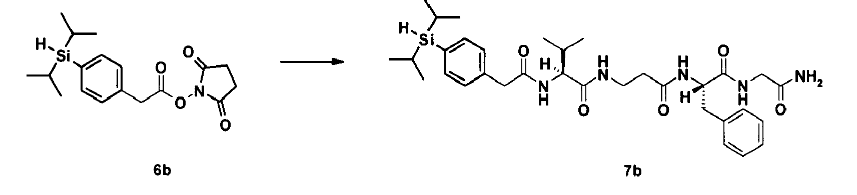 MS (ES+): 624.27

### PART F2

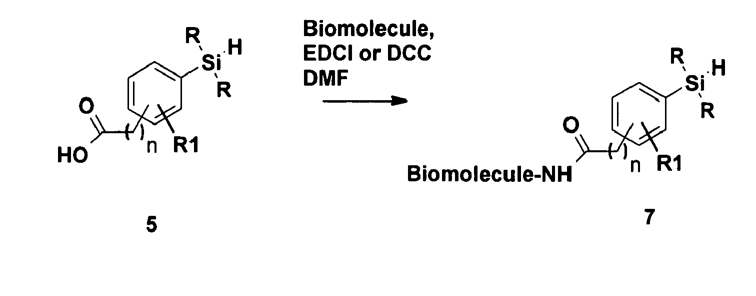

### General Method

0.05 mmol biomolecule or resin bound protected peptide suspended in 2 ml DMF was treated with 0.15 mmol silane acid 5, 57 mg (0.15 mmol) HBTU, 23 mg (0.15 mmol) HOBT and 26 µl (0.15 mmol) diisopropyl ethyl amine for 12h. The reaction mixture was filtered and the resin was washed with DMF and dichloromethane. After cleavage from the resin with 1 mL of a mixture of 85% TFA, 5% water, 5% phenol, and 5% triisopropylsilane, the product was precipitated with MTBE and purified by HPLC to give 13% of 7.
4-(Diisopropylsilanyl)-phenyl)-acetyl-Val-ßAla-Phe-Gly-NH₂ 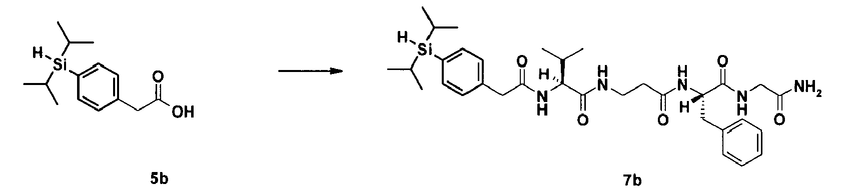 MS (ES+): 624.27

### PART G

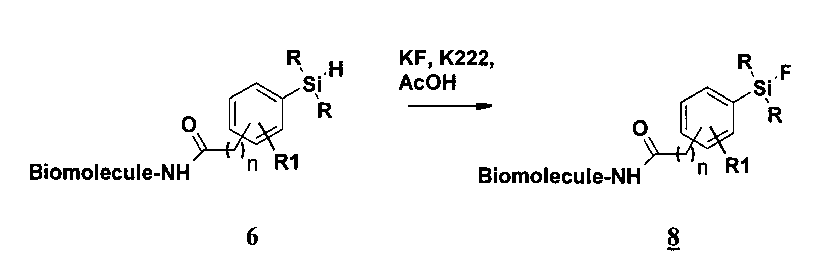

### General Method

2 mg (3.21 µM) silane 6 were solved in 320 µl THF and treated with 0.19 mg (3.21 µM) KF, 1.21 mg (3.21 µM) K222, 0.44 mg (3.21 µM) K₂CO₃ and 0.55 µl acetic acid. The reaction mixture was stirred at 50° for 30 min and followed by HPLC to give 60-90% conversion to 8.
[4-(fluoro-diisopropyl-silanyl)-phenyl]-acetic acid - Val-ßAla-Phe-Gly-amide 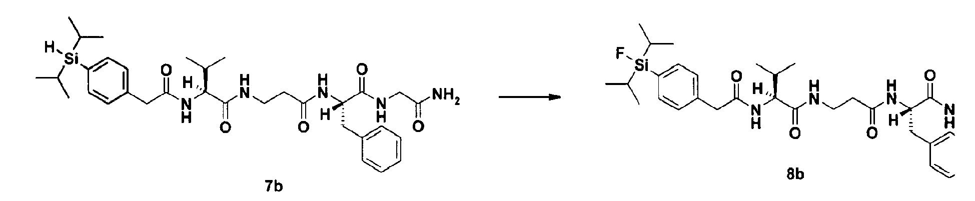 MS (ES+): 642.12

### EXAMPLE II

### PART A

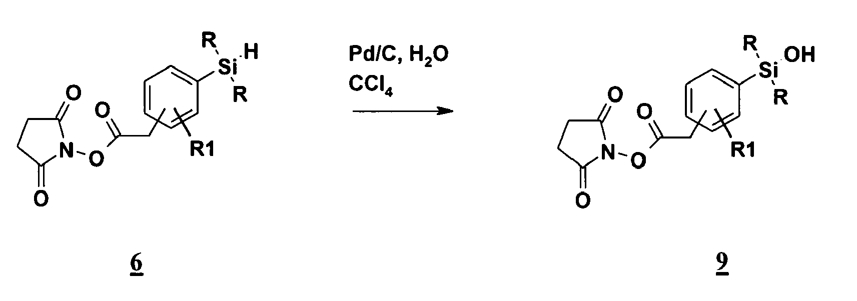

### General Method

To a solution of 399 µmol **6** in 1.68 mL tetrachloromethane was added 7.27 mg palladium (10% on charcoal) and the mixture stirred for 16 hours at 23°C. 72 µL water were added and stirring was continued for additional 75 hours at 23°C. Sodium sulfate was added and after filtration the solvent was evaporated. The crude product was purified by chromatography on silica gel to give 64-84% of **9**.
4-(Hydroxy-diisopropyl-silanyl)-benzoic acid 2,5-dioxo-pyrrolidin-1-yl ester 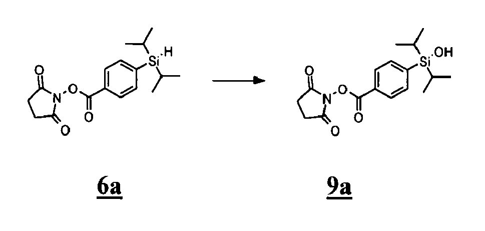 ¹H-NMR (CDCl₃): δ = 8.11 (2H), 7.71 (2H), 2.92 (4H), 1.87 (1H), 1.24 (2H), 1.05 (6H), 0.96 (6H) ppm.
[4-(Hydroxy-diisopropyl-silanyl)-phenyl]-acetic acid 2,5-dioxo-pyrrolidin-1-yl ester 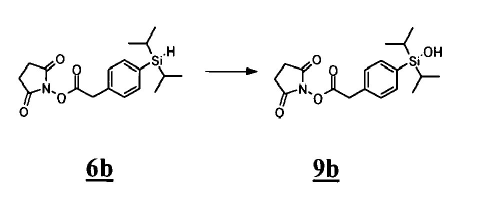 ¹H-NMR (CDCl₃): δ = 7.55 (2H), 7.35 (2H), 3.94 (2H), 2.83 (4H), 1.80 (1H), 1.21
(2H), 1.05 (6H), 0.97 (6H) ppm.
3-[3-(Hydroxy-diisopropyl-silanyl)-phenyl]-propionic acid 2,5-dioxo-pyrrolidin-1-yl ester 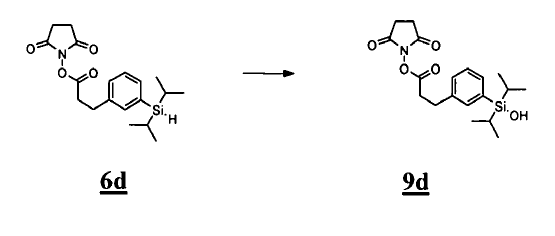 ¹H-NMR (CDCl₃): δ = 7.45 (1H), 7.41 (1H), 7.31 (1H), 7.25 (1H), 3.09 (2H), 2.93 (2H), 2.84 (4H), 1.21 (2H), 1.06 (6H), 0.98 (6H) ppm.
4-(Hydroxy-diisobutyl-silanyl)-benzoic acid 2,5-dioxo-pyrrolidin-1-yl ester 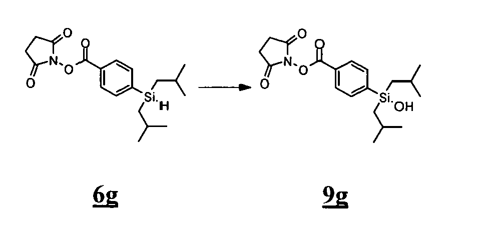 ¹H-NMR (CDCl₃): δ = 8.11 (2H), 7.73 (2H), 2.91 (4H), 1.79 (2H), 1.60 (1H), 0.92 (6H), 0.89 (6H), 0.86 (4H) ppm.
[4-(Hydroxy-diisobutyl-silanyl)-phenyl]-acetic acid 2,5-dioxo-pyrrolidin-1-yl ester 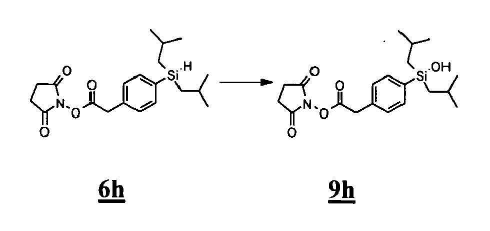 ¹H-NMR (CDCl₃): δ = 7.57 (2H), 7.34 (2H), 3.94 (2H), 2.83 (4H), 1.82 (2H), 0.93 (6H), 0.91 (6H), 0.84 (4H) ppm.

### PART B

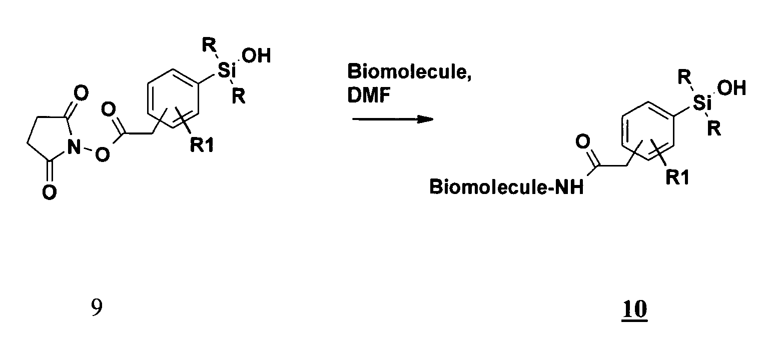

### General Method

0.05 mmol biomolecule or resin bound protected peptide suspended in 2 ml DMF was treated with 0.15 mmol silanol active ester 9 for 12h. The reaction mixture was filtered and in case of the resin bound product, the resin was washed with DMF and dichloro-methane. After cleavage from the resin with 1 ml of a mixture of 85% TFA, 5% water, 5% phenol, and 5% triisopropylsilane, the product was precipitated with MTBE and purified by HPLC to give 12-45% of 10. Reaction mixtures with soluble biomolecules were evaporated and the residue was purified by column chromatography to give 68-80% of 10.
4-(Hydroxy-diisopropyl-silanyl)-phenylacetyl-Val-ßAla-Phe-Gly-NH₂ 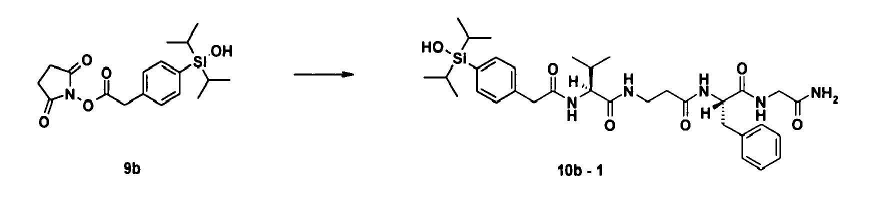 MS (ES+): 640.29
3-(Hydroxy-diisopropyl-silanyl)- phenylpropionyl-Val-ßAla-Phe-Gly-NH₂ 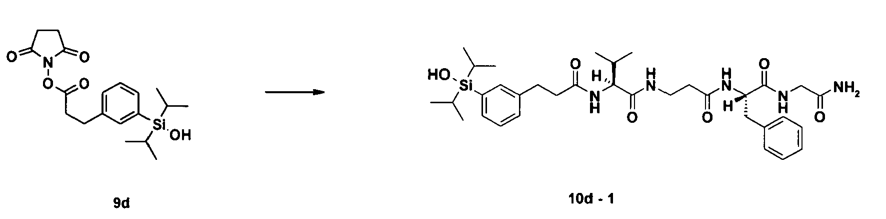 MS (ES+): 654.11

### PART C

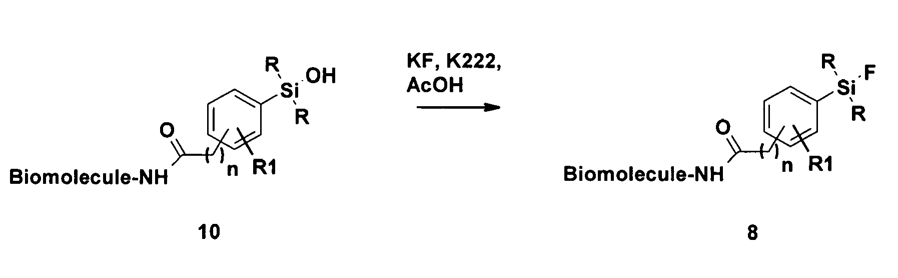

### General Method

6.12 µM silanol 10 were solved in 600 µl THF and treated with 1.42 mg (24.47 µM) KF, 9.21 mg (24.47 µM) K222, 1.69 mg (12.23 µM) K₂CO₃ and 4.2 µl acetic acid. The reaction mixture was stirred at 50° for 30 min and followed by HPLC purification to give 30 - 41 % of 8.
4-(Fluoro-diisopropyl-silanyl)-phenylacetyl-Val-ßAla-Phe-Gly-NH₂ 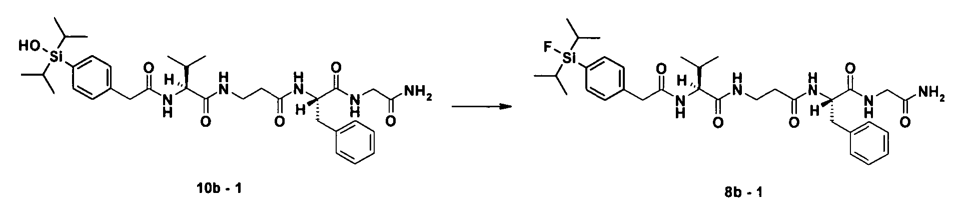 MS (ES+): 641.99
3-(Fluoro-diisopropyl-silanyl)- phenylpropionyl-Val-ßAla-Phe-Gly-NH₂ 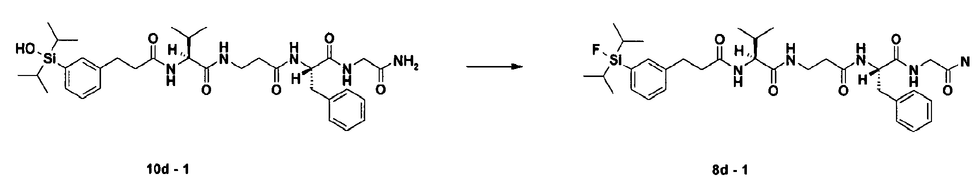 MS (ES+): 655.89

### Fluorination of model compounds

### Silanes

### General Method

To a solution of 120 µmol silane in 0.49 mL THF were added 31.7 mg 18-crown-6, 8.3 mg potassium carbonate, 20.6 µL acetic acid and 6.96 mg potassium fluoride. The mixture was stirred 2.5 hours at 23°C and 1.5 hours at 50°C. After purification by chromatography on silica gel 49-87% of the title compounds were obtained as an oil. [4-(Fluoro-diisopropyl-silanyl)-phenyl]-acetic acid 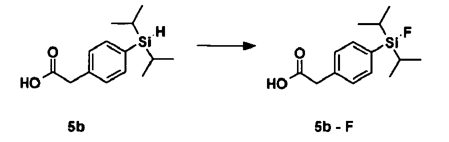 ¹H-NMR (CDCl₃): δ = 7.52 (2H), 7.32 (2H), 3.67 (2H), 1.26 (2H), 1.07 (6H), 1.01 (6H) ppm. ¹⁹F-NMR (CDCl₃): δ = -187.0 ppm.
2-[4-(Fluoro-diisopropyl-silanyl)-phenyl]-ethanol 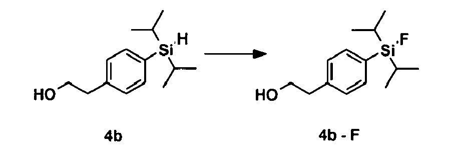 ¹H-NMR (CDCl₃): δ = 7.50 (2H), 7.27 (2H), 3.39 (2H), 2.89 (2H), 1.48-1.36 (1H), 1.27 (2H), 1.08 (6H), 1.02 (6H) ppm. ¹⁹F-NMR (CDCl₃): δ = -187.0 ppm.
(RS)-Fluoro-diisopropyl- {4-[2-(tetrahydro-pyran-2-yloxy)-ethyl]-phenyl}-silane 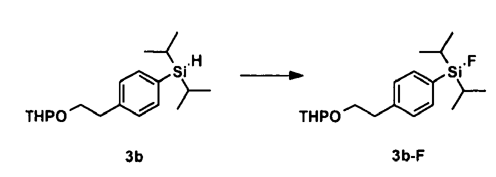 ¹H-NMR (CDCl₃): δ = 7.47 (2H), 7.28 (2H), 4.60 (1H), 3.95 (1H), 3.70-3.61 (2H), 3.42 (1H), 2.93 (2H), 1.98-1.40 (6H), 1.26 (2H), 1.07 (6H), 1.00 (6H) ppm. ¹⁹F-NMR (CDCl₃): δ = -187.2 ppm.
(RS)-Fluoro-diisopropyl-[4-(tetrahydro-pyran-2-yloxymethyl)-phenyl]-silane 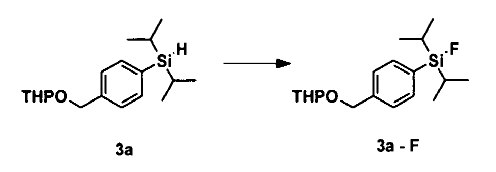 ¹H-NMR (CDCl₃):δ = 7.53 (2H), 7.40 (2H), 4.82 (1H), 4.74 (1H), 4.51 (1H), 3.93 (1H), 3.57 (1H), 1.93-1.52 (6H), 1.27 (2H), 1.08 (6H), 1.01 (6H) ppm. ¹⁹F-NMR (CDCI₃): δ = -187.2 ppm.
4-(Fluoro-diisopropyl-silanyl)-benzoic acid 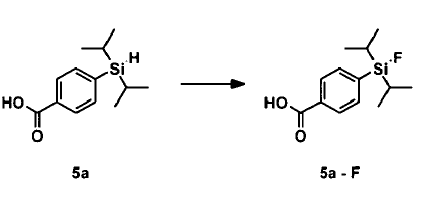 ¹H-NMR (CDCl₃): δ = 8.11 (2H), 7.68 (2H), 1.31 (2H), 1.09 (6H), 1.02 (6H) ppm. ¹⁹F-NMR (CDCl₃): δ = -187.5 ppm.
[4-(Fluoro-diisopropyl-silanyl)-phenyl]-methanol 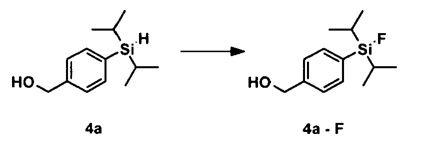 ¹H-NMR (CDCl₃): δ = 7.55 (2H), 7.40 (2H), 4.72 (2H), 1.65 (1H), 1.27 (2H), 1.08 (6H), 1.01 (6H) ppm.¹⁹F-NMR (CDCl₃): δ = -187.3 ppm.
(RS)-Fluoro-diisopropyl-{4-[3-(tetrahydro-pyran-2-yloxy)-propyl]-phenyl}-silane 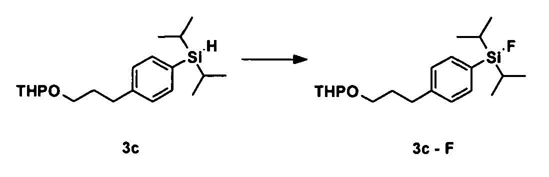 ¹H-NMR (CDCl₃): δ = 7.46 (2H), 7.23 (2H), 4.59 (1H), 3.87 (1H), 3.78 (1H), 3.50 (1H), 3.42 (1H), 2.72 (2H), 1.95 (2H), 1.84 (1H), 1.72 (1H), 1.64-1.48 (4H), 1.26 (2H), 1.08 (6H), 1.01 (6H) ppm. ¹⁹F-NMR (CDCl₃): δ = -187.0 ppm.
3-[4-(Fluoro-diisopropyl-silanyl)-phenyl]-propan-1-ol 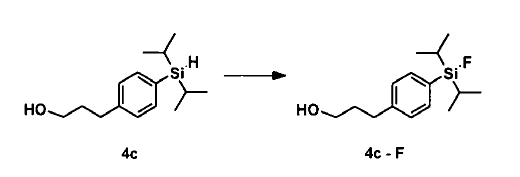 ¹H-NMR (CDCl₃): δ = 7.47 (2H), 7.23 (2H), 3.69 (2H), 2.73 (2H), 1.92 (2H), 1.59 (1H), 1.26 (2H), 1.08 (6H), 1.02 (6H) ppm. ¹⁹F-NMR (CDCl₃): δ = -187.0 ppm.
3-[4-(Fluoro-diisopropyl-silanyl)-phenyl]-propionic acid 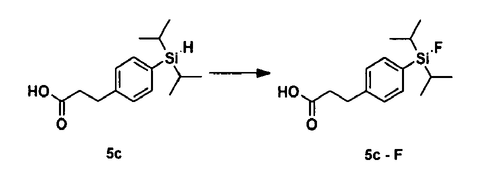 ¹H-NMR (CDCl₃): δ = 7.48 (2H), 7.24 (2H), 2.98 (2H), 2.71 (2H), 1.24 (2H), 1.08 (6H), 1.01 (6H) ppm. ¹⁹F-NMR (CDCl₃): δ = -187.0 ppm.
(RS)-Fluoro-diisopropyl-{3-[3-(tetrahydro-pyran-2-yloxy)-propyl]-phenyl}-silane 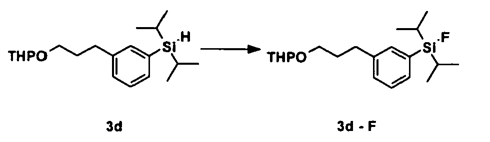 ¹H-NMR (CDCI₃): δ = 7.37-7.25 (4H), 4.57 (1H), 3.87 (1H), 3.78 (1H), 3.50 (1H), 3.40 (1H), 2.72 (2H), 1.93 (2H), 1.83 (1H), 1.72 (1H), 1.63-1.49 (4H), 1.26 (2H), 1.08 (6H), 1.01 (6H) ppm. ¹⁹F-NMR (CDCI₃): δ = -187.3 ppm.
3-[3-(Fluoro-diisopropyl-silanyl)-phenyl]-propan-1-ol 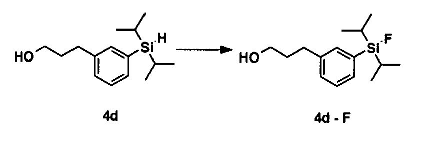 ¹H-NMR (CDCl₃): δ = 7.39-7.26 (4H), 3.70 (2H), 2.73 (2H), 1.91 (2H), 1.28 (2H), 1.09 (6H), 1.02 (6H) ppm. ¹⁹F-NMR (CDCl₃): δ = -187.3 ppm.
3-[3-(Fluoro-diisopropyl-silanyl)-phenyl]-propionic acid 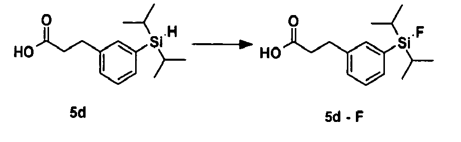 ¹H-NMR (CDCl₃): δ = 7.42-7.25 (4H), 2.98 (2H), 2.69 (2H), 1.27 (2H), 1.07 (6H), 1.01 (6H) ppm. ¹⁹F-NMR (CDCl₃): δ = -187.2 ppm.
(RS)-Fluoro-diisopropyl- {3-[2-(tetrahydro-pyran-2-yloxy)-ethyl]-phenyl}-silane 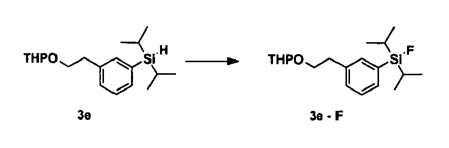 ¹H-NMR (CDCl₃): δ = 7.42 (1H), 7.38 (1H), 7.31 (2H), 4.59 (1H), 3.95 (1H), 3.71 (1H), 3.61 (1H), 3.44 (1H), 2.92 (2H), 1.80 (1H), 1.68 (1H), 1.62-1.42 (4H), 1.26 (2H), 1.08 (6H), 1.01 (6H) ppm. ¹⁹F-NMR (CDCl₃): δ = -187.4 ppm.
2-[3-(Fluoro-diisopropyl-silanyl)-phenyl]-ethanol 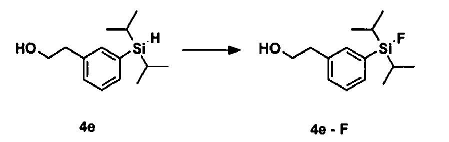 ¹H-NMR (CDCl₃): δ = 7.41 (2H), 7.35 (1H), 7.30 (1H), 3.87 (2H), 2.89 (2H), 1.39 (1H), 1.27 (2H), 1.08 (6H), 1.02 (6H) ppm. ¹⁹F-NMR (CDCl₃): δ = -187.1 ppm. [3-(Fluoro-diisopropyl-silanyl)-phenyl]-acetic acid 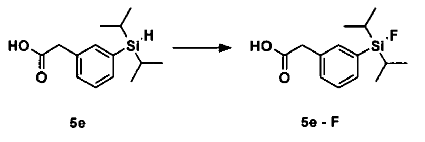 ¹H-NMR (CDCl₃): δ = 7.48-7.31 (4H), 3.38 (2H), 1.28 (2H), 1.08 (6H), 1.02 (6H) ppm. ¹⁹F-NMR (CDCl₃): δ = -187.1 ppm.
[4-(Fluoro-diisobutyl-silanyl)-phenyl]-acetic acid 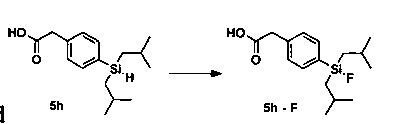 ¹H-NMR (CDCI₃): δ = 7.53 (2H), 7.32 (2H), 3.67 (2H), 1.85 (2H), 0.96-0.86 (16H) ppm. ⁹F-NMR (CDCI₃): δ = -169.6 ppm.
(RS)-Fluoro-diisobutyl-{4-[2-(tetrahydro-pyran-2-yloxy)-ethyl]-phenyl}-silane 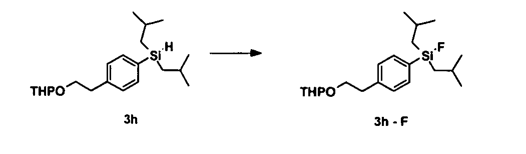 ¹H-NMR (CDCl₃): δ = 7.48 (2H), 7.27 (2H), 4.59 (1H), 3.95 (1H), 3.69 (1H), 3.63 (1H), 3.43 (1H), 2.92 (2H), 1.84 (2H), 1.80 (1H), 1.69 (1H), 1.63-1.43 (4H), 0.98-0.85 (16H) ppm. ¹⁹F-NMR (CDCI₃): δ = -169.6 ppm.
(RS)-Fluoro-diisobutyl-[4-(tetrahydro-pyran-2-yloxymethyl)-phenyl]-silane 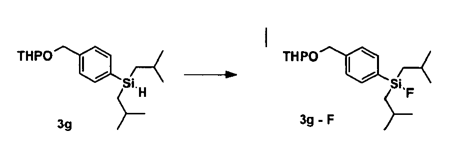 ¹H-NMR (CDCl₃): δ = 7.55 (2H), 7.40 (2H), 4.81 (1H), 4.73 (1H), 4.51 (1H), 3.93 (1H), 3.56 (1H), 1.92-1.51 (8H), 0.98-0.85 (16H) ppm. ¹⁹F-NMR (CDCl₃): δ = -169.7 ppm.
4-(Fluoro-diisobutyl-silanyl)-benzoic acid 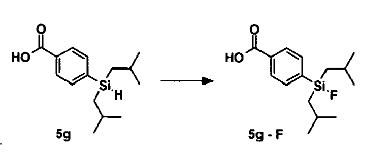 ¹H-NMR (CDCI₃): δ = 8.12 (2H), 7.69 (2H), 1.85 (2H), 0.98-0.83 (16H) ppm.¹⁹F-NMR (CDCl₃): δ = -170.4 ppm.
(RS)-Fluoro-diphenyl-{4-[2-(tetrahydro-pyran-2-yloxy)-ethyl]-phenyl}-silane 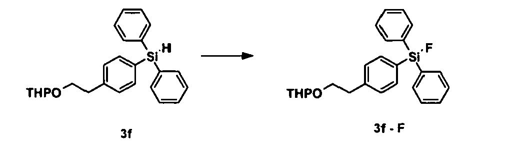 ¹H-NMR (CDC1₃): δ = 7.68-7.30 (14H), 4.64 (1H), 3.99 (1H), 3.77 (1H), 3.66 (1H), 3.48 (1H), 2.97 (2H), 1.88-1.60 (6H) ppm. ¹⁹F-NMR (CDCl₃): δ= -169.4 ppm.

### Silanols, preparation of model compounds

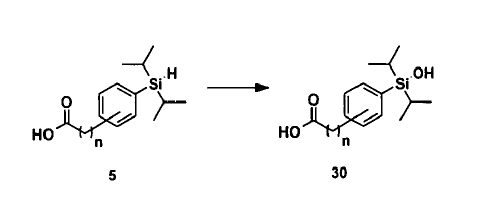

### General Method

To a solution of 399 µmol **5** (Example I, Part D) in 1.68 mL tetrachloro-methane was added 7.27 mg Palladium (10% on charcoal) and the mixture stirred for 16 hours at 23°C. 72 µL water were added and stirring was continued for additional 75 hours at 23°C. Sodium sulfate was added and after filtration the solvent was evaporated. The crude product was purified by chromatography on silica gel to give 68-75% of the title compound **30** as an oil.
4-(Hydroxy-diisopropyl-silanyl)-benzoic acid 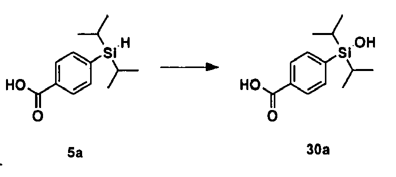 1H-NMR (CDCl3): δ = 8,09 (2H), 7.69 (2H), 1.25 (2H), 1.06 (6H), 0.98 (6H) ppm.
4-(Hydroxy-diisopropyl-silanyl)-phenyl]-acetic acid 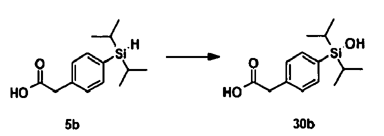 ¹H-NMR (CDCl₃): δ = 7.51 (2H), 7.29 (2H), 5.20-4.50 (2H), 3.65 (2H), 1.20 (3H), 1.04 (6H), 0.96 (6H) ppm.
4-(Hydroxy-diisobutyl-silanyl)-benzoic acid 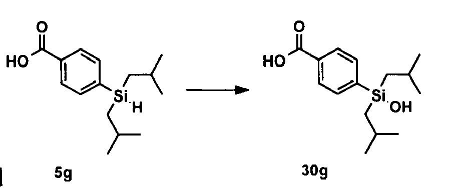 ¹H-NMR (CDCl₃): δ = 8.08 (2H), 7.70 (2H), 1.82 (2H), 0.93 (6H), 0.91 (6H), 0.88 (4H) ppm.
[4-(Hydroxy-diisobutyl-silanyl)-phenyl]-acetic acid 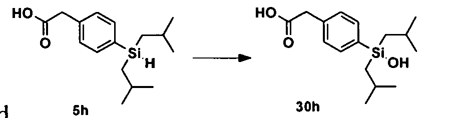 ¹H-NMR (CDCI₃): δ = 7.54 (2H), 7.29 (2H), 3.65 (2H), 1.82 (2H), 0.93 (6H), 0.91 (6H), 0.84 (4H) ppm.

### Silanols, fluorination

To a solution of 139 µmol silanol 30 in 0.57 mL THF were added 36.7 mg 18-crown-6, 9.6 mg potassium carbonate, 23.8 µL acetic acid and 8.07 mg potassium fluoride. The mixture was stirred at 50°C for 1.5 hours and purified by chromatography on silica gel to yield 17 mg 46-60% of the title compounds 30-F as an oil.
4-(Fluoro-diisopropyl-silanyl)-benzoic acid 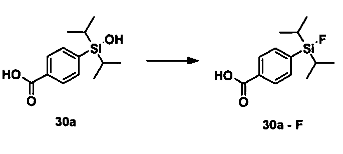 ¹H-NMR (CDCl₃): δ= 8.11 (2H), 7.68 (2H), 1.31 (2H), 1.09 (6H), 1.02 (6H) ppm. ¹⁹F-NMR (CDCl₃): δ= -187.5 ppm.
[4-(Fluoro-diisopropyl-silanyl)-phenyl]-acetic acid 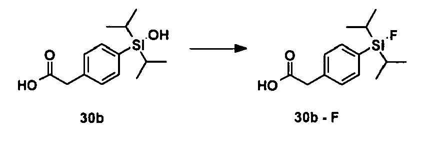 ¹H-NMR (CDCl₃): δ = 7.52 (2H), 7.32 (2H), 3.67 (2H), 1.26 (2H), 1.07 (6H), 1.01 (6H) ppm. ¹⁹F-NMR (CDCl₃): δ= -187.0 ppm.
4-(Fluoro-diisobutyl-silanyl)-benzoic acid 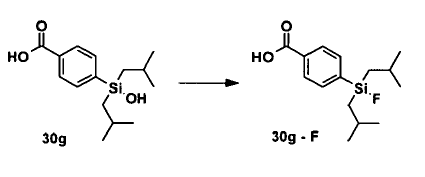 ¹H-NMR (CDCI₃): δ= 8.12 (2H), 7.69 (2H), 1.85 (2H), 0.98-0.83 (16H) ppm.¹⁹F-NMR (CDCI₃): δ= -170.4 ppm.
[4-(Fluoro-diisobutyl-silanyl)-phenyl]-acetic acid 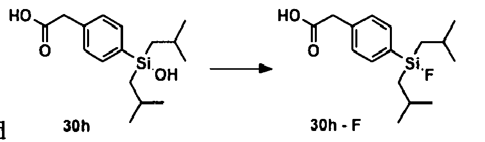 ¹H-NMR (CDCI₃): δ= 7.53 (2H), 7.32 (2H), 3.67 (2H), 1.85 (2H), 0.96-0.86 (16H) ppm. ¹⁹F-NMR (CDCI₃): δ= -169.6 ppm.

### Example III

### Part A

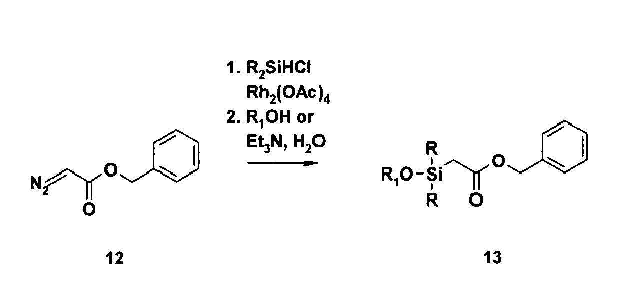

(Di-tert-butyl-hydroxy-silanyl)-acetic acid benzyl ester

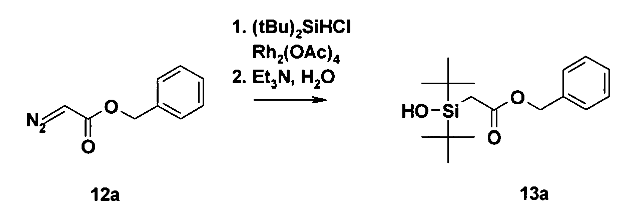

A solution of benzyl diazoacetate 12a (4.00mmol, 705mg) in anhydrous dichloro-methane (1ml) was added very slowly at room temperature, using a syringe pump (2mmol/h), to a solution of di-*tert*-butylchlorosilane (4.00mmol, 0.82ml) and Rh₂(OAc)₄ (0.012mmol, 5.7mg) in anhydrous dichloromethane (1.5ml). The mixture was cooled to 0°C. Then a solution of triethylamine (5.00mmol, 0.70ml) in anhydrous dichloromethane (1ml) and water (5.00mmol, 0.09ml) were added successively dropwise. The suspension was stirred for 3h and then treated at 0°C with a saturated solution of NaHCO₃ and the organic layer was separated. The aqueous layer was extracted with dichloromethane and the combined extracts were washed with brine, dried (MgSO₄) and evaporated in vacuo. The residue was purified by column chromatography (pentane-EtOAc 19:1) to give 835mg benzyl 2-(di-*tert*-butyl(hydroxy)silyl)acetate 13a (68%) as a white solid.

### Part C

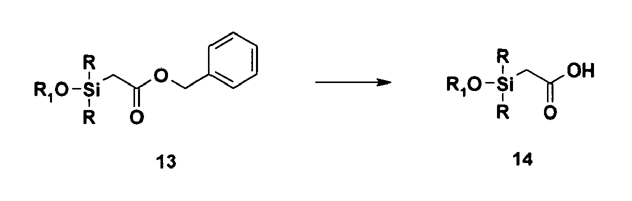

(Di-tert-butyl-hydroxy-silanyl)-acetic acid

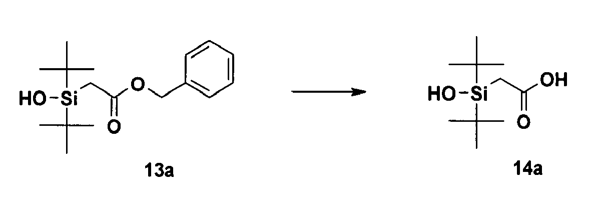

Into an argon flushed flask 10% Pd/C (30mg) was added. Benzyl 2-(di-*tert-*butyl(hydroxy)silyl)acetate 13a (2.70mmol, 834mg) in EtOAc (10ml) was then added. The argon atmosphere was then replaced by hydrogen. The reaction mixture was stirred overnight at room temperature and then filtered over Celite (eluent: EtOAc). Evaporation of the solvent gave 552mg 2-(di-*tert*-butyl(hydroxy)silyl)acetic acid 14a (94%). ¹H-NMR δ 1.05 (s, 18H, Si(tBu)₂), 2.04 (s, 2H, SiCH₂). ¹³C-NMR (CDCl₃, 100MHz): δ 20.7 (SiCH₂), 21.0 (SiC), 27.4 (CH₃), 178.5 (C=O). MS (ESI positive): 219.2 [M+H].

### Part D

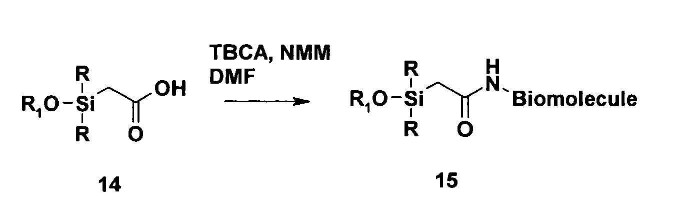

(Di-tert-butyl-hydroxy-silanyl)-acetyl- Val-ßAla-Phe-Gly-NH₂ 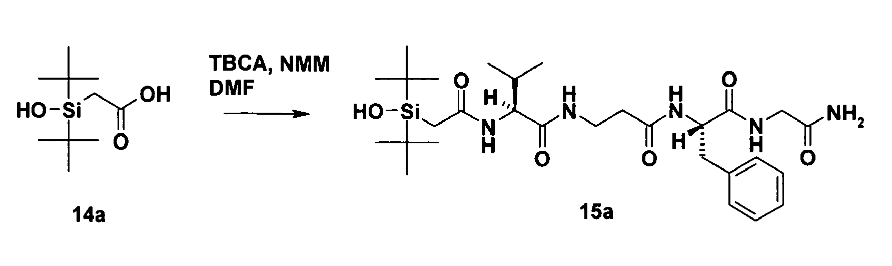 30 mg Di-tert-butyl-hydroxy-silanyl)-acetic acid 14a (137 µM), 45 mg (137 µM) TBCA and 14 mg (137 µM) NMM were solved in 1ml DMF and stirred for 30 min. The reaction mixture was added to 69 µM of the resin bound protected peptide suspended in 2 ml DMF for 14h. The resin was filtered, washed with DMF and dichloromethane. After cleavage from the resin with 1 ml of a mixture of 85% TFA, 5% water, 5% phenol, and 5% triisopropylsilane, the product was precipitated with MTBE and purified by HPLC to give 33% of 15a.

### Part E

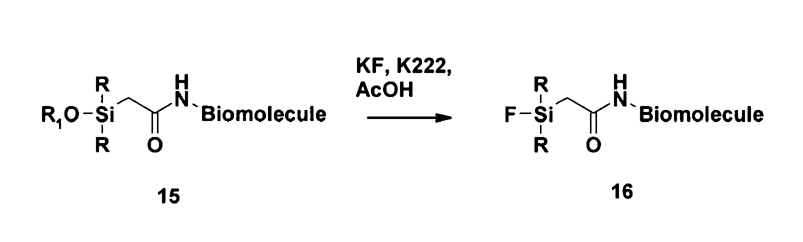

(Di-tert-butyl-fluoro-silanyl)-acetyl- Val-ßAla-Phe-Gly-NH₂

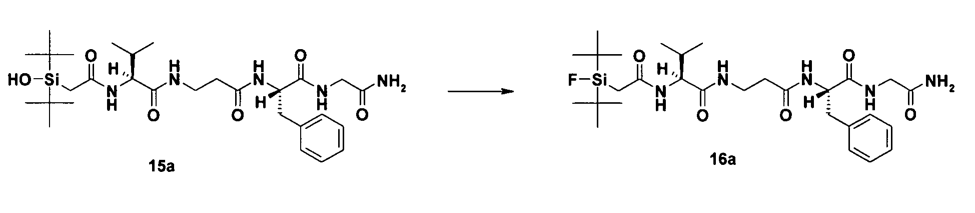

### Fluorination model compound

### Benzyl 2-(di-tert-butylfluorosilyl)acetate

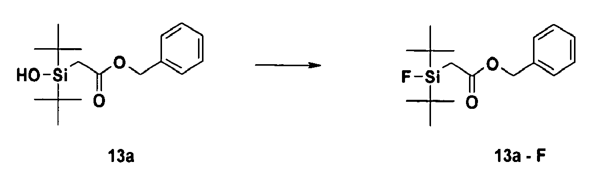

To benzyl 2-(di-*tert*-butyl(hydroxy)silyl)acetate 13a (2.70mmol, 833mg) and 4-methoxysalicylaldehyde (2.70mmol, 411mg) in anhydrous dichloromethane (30ml) was added at room temperature boron trifluoride diethyl etherate (5.40mmol, 0.68ml). After stirring the reaction mixture at room temperature for 4h, the solution was hydrolyzed with water (5ml) and vigourously stirred for 10min. The organic layer was washed with brine, dried over MgSO₄, filtered and solvents removed by evaporation under vacuum. The crude product was purified by column chromatography (pentane-EtOAc 99: 1) to give 740mg benzyl 2-(di-*tert*-butylfluorosilyl)acetate (88%) as a colorless oil. ¹H-NMR δ 1.07 (s, 18H, Si(tBu)₂), 2.18 (d, 2H, SiCH₂), 5.10 (s, 2H, COCH₂), 7.29-7.38 (m, 5H, Ar-H). ¹³C-NMR (CDCl₃, 100MHz): δ 20.8 (d, SiC), 20.9 (d, SiC), 27.0 (CH₃), 66.5 (OCH₂), 128.2 (Ar-CH), 128.5 (Ar-CH), 128.6 (Ar-CH), 136.0 (Ar-C), 171.5 (d, C=O). ¹⁹F-NMR (CDCl₃, 376MHz): δ -181.0 (m). MS (ESI positive): 311.1 [M+H].

### Example IV

### 4-[3-(Ethoxy-diisopropyl-silanyl)-propylcarbamoyl]-butyric acid

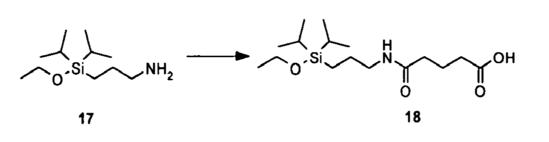

The solution of 500 mg (2.3 mmol) 3-(ethoxy-diisopropyl-silanyl)-propylamine**17** in 24 ml dichloro-methane was cooled to 3°C, 276 mg dihydro-pyran-2,6-dione added and the mixture was stirred for 30 minutes. The solvent was removed and 776 mg of the title compound **18** were isolated without further purification. ¹H-NMR (CDCI₃): δ = 6.84-6.27 (1H), 6.01 (1H), 3.77 (2H), 3.28 (2H), 2.46 (2H), 2.32 (2H), 2.01 (2H), 1.64 (2H), 1.24 (3H), 1.06 (14H), 0.66 (2H) ppm.

### 4-[3-(Ethoxy-diisopropyl-silanyl)-propylcarbamoyl]-butyric acid 2,5-dioxo-pyrrolidin-1-yl ester

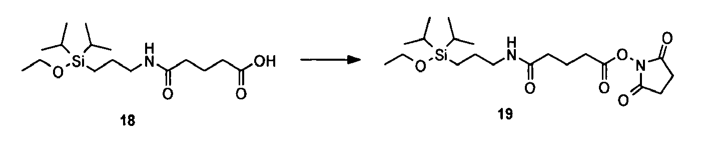

To 300 mg (0.9 mmol) 4-[3-(ethoxy-diisopropyl-silanyl)-propylcarbamoyl]-butyric acid 18, solved in 9 mL dichloro-methane were added 114 mg N-hydroxy-succinimide, 189 mg (3-Dimethylamino-propyl)-ethyl-carbodiimide hydrochloride and the mixture was stirred for 16 hours at 23°C. After addition of water and extraction with dichloro-methane the combined organic extracts were dried over sodium sulfate. After filtration and solvent evaporation the crude product was purified by chromatography on silica gel to give 172 mg (44%) of 19. ¹H-NMR (CDCl₃): δ = 6.10 (1H), 3.76 (2H), 3.28 (2H), 2.88 (4H), 2.72 (2H), 2.33 (2H), 2.15 (2H), 1.62 (2H), 1.22 (3H), 1.05 (14H), 0.66 (2H) ppm.

### Part A

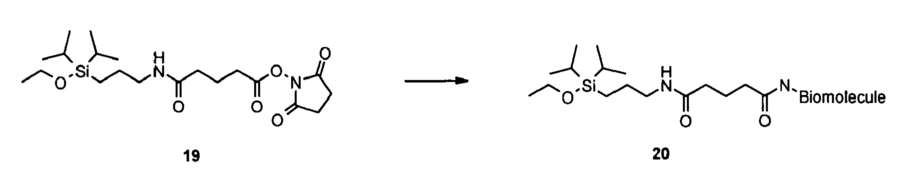

### 4-[3-(Ethoxy-diisopropyl-silanyl)-propylcarbamoyll-butyric-Val-ßAla-Phe-Gly-NH₂

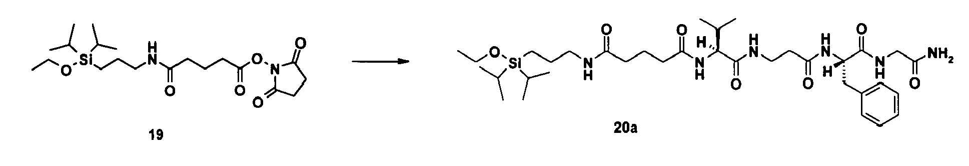

MS(ES+): 704.99

### Part B

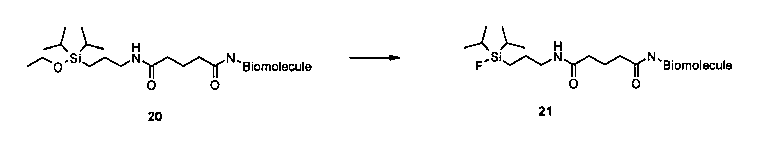

### Model compound of example 4

### N-[3-(Ethoxy-diisopropyl-silanyl)-propyl]-benzamide

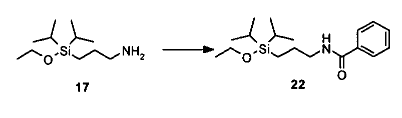

To the solution of 1.0 g 3-(ethoxy-diisopropyl-silanyl)-propylamine **17** in 10 mL dichloro-methane were added at 3°C 535 µL benzoyl chloride, 638 µL triethyl-amine and the mixture was stirred for 1.5 hours. The mixture was poured into water, extracted with dichloro-methane and the combined organic extracts were dried over sodium sulfate. After filtration und evaporation of the solvent 1.44g (97%) of the title compound **22** were isolated as an oil.
¹H-NMR (CDCl₃): δ = 7.78 (2H), 7.50 (1H), 7.43 (2H), 6.40 (1H), 3.74 (2H), 3.46 (2H), 1.74 (2H), 1.19 (3H), 1.04 (14H), 0.71 (2H) ppm.

### Fluorination of model compound

### N-[3-(Fluoro-diisopropyl-silanyl)-propyl]-benzamide

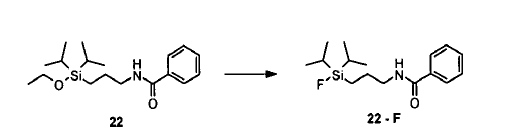

50 mg (156 µmol) N-[3-(ethoxy-diisopropyl-silanyl)-propyl]-benzamide 22 was transformed in analogy to silanes to yield after isolation and purification 28 mg (61 %) of the title compound 22 - F as an oil.
¹H-NMR (CDCI₃): δ = 7.80 (2H), 7.54 (1H), 7.47 (2H), 3.50 (2H), 1.79 (2H), 1.68 (1H), 1.12-1.03 (14H), 0.81 (2H) ppm. ¹⁹F-NMR (CDCI₃): δ= -181.3 ppm.

### Example V

### Pent-4-enoic acid 2,5-dioxo-pyrrolidin-1-yl ester

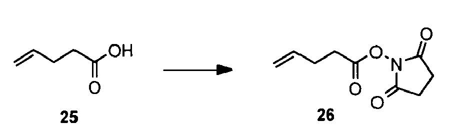

To a solution of 5 g (49.9 mmol) pent-4-enoic acid **25** in 250 mL dichloromethane were added 6.32 g N-hydroxy-succinimide, 10.48 g mg (3-Dimethylamino-propyl)-ethylcarbodiimide hydrochloride and the mixture was stirred for 16 hours at 23°C. After addition of water and extraction with dichloro-methane the combined organic extracts were dried over sodium sulfate. After filtration and solvent evaporation the crude product was purified by chromatography on silica gel to give 8.8 g (89%) of the title compound **26** as an oil.
¹H-NMR (CDCI₃): δ = 5.89 (1H), 5.17 (1H), 5.12 (1H), 2.87 (4H), 2.75 (2H), 2.53 (2H) ppm.

### Part A

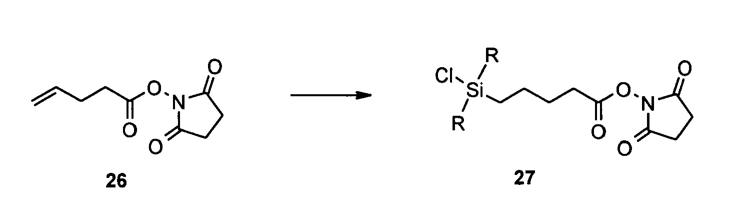

### 5-(Chloro-diisobutyl-silanyl)-pentanoic acid 2,5-dioxo-pyrrolidin-1-yl ester

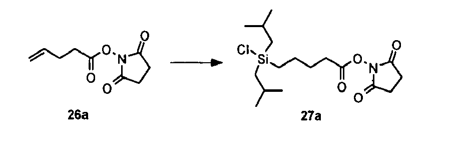

500 mg (2.54 mmol) pent-4-enoic acid 2,5-dioxo-pyrrolidin-1-yl ester **26a** were solved in 2 mL THF and 907 mg chloro-diisobutyl-silane followed by a catalytic amount of Karstedts catalyst were added. After 2 hours of stirring the mixture containing title compound 27a was used directly for the next reaction.

### Part B

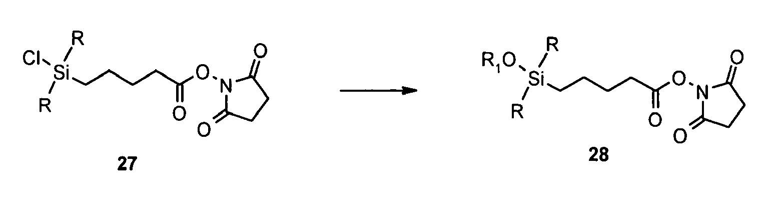

### 5-[Diisobutyl-(4-phenyl-butoxy)-silanyl]-pentanoic acid 2,5-dioxo-pyrrolidin-1-yl ester

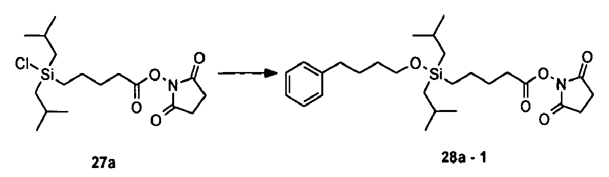

To the reaction mixture containing 5-(chloro-diisobutyl-silanyl)-pentanoic acid 2,5-dioxo-pyrrolidin-1-yl ester **27a** (2.54 mmol at maximum) were added at 3°C 2 mL THF, 781 µL 4-phenyl-butan-1-ol 763 µL 1,5-diaza-bicyclo[3.3.3]undecane. After 2 hours the mixture was pured into water and extracted with ethyl acetate. The combined organic extracts were washed with brine and dried over sodium sulfate. After filtration and removal of the solvent the crude product was purified by chromatography on silica gel to give 762 mg (61%) of the title compound **28a** as an oil.
¹H-NMR (CDCl₃): δ = 7.35-7.28 (3H), 7.24-7.19 (2H), 3.63 (2H), 2.85 (4H), 2.66 (4H), 1.89-1.46 (12H), 0.99 (14H), 0.67 (2H) ppm.
5-[(4-Polystyrene-methoxy-benzyloxy)-diisobutyl-silanyl]-pentanoic acid 2,5-dioxo-pyrrolidin-1-yl ester

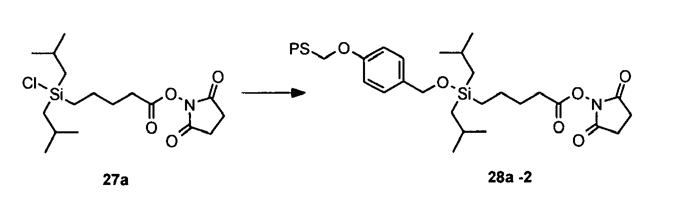

To a suspension of 566 mg Wang resin in 10 mL dichloro-methane was added the reaction mixture containing 5-(chloro-diisobutyl-silanyl)-pentanoic acid 2,5-dioxo-pyrrolidin-1-yl ester **27a** (2.54 mmol at maximum) 398 µL 1,5-diaza-bicyclo[3.3.3]undecane. The mixture was stirred for 2.5 hours at 23°C and after filtration the residue was washed with dichloro-methane and dried. 713 mg (68%) of the title compound **28a-2** were isolated as solid.

### 5-(Polystyrene-methoxy-diisobutyl-silanyl)-pentanoic acid 2,5-dioxo-pyrrolidin-1-yl ester

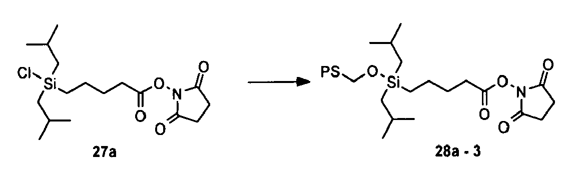

To a suspension of 576 mg hydroxymethyl-polystyrene resin in 5 mL dichloro-methane was added the reaction mixture containing 5-(chloro-diisobutyl-silanyl)-pentanoic acid 2,5-dioxo-pyrrolidin-1-yl ester **27a** (2.54 mmol at maximum) and 398 µL 1,5-diaza-bicyclo[3.3.3]undecane. The mixture was stirred for 2.5 hours at 23°C and after filtration the residue was washed with dichloro-methane and dried. 762 mg (62%) of the title compound **28a - 3** were isolated as solid.

### 5-(Polystyrene-ethoxy-diisobutyl-silanyl)-pentanoic acid 2,5-dioxo-pyrrolidin-1-yl ester

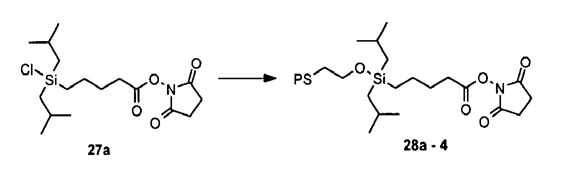

To a suspension of 576 mg 2-hydroxyethyl-polystyrene resin in 10 mL dichloro-methane was added the reaction mixture containing 5-(chloro-diisobutyl-silanyl)-pentanoic acid 2,5-dioxo-pyrrolidin-1-yl ester **27a** (2.54 mmol at maximum) and 398 µL 1,5-diaza-bicyclo[3.3.3]undecane. The mixture was stirred for 2.5 hours at 23°C and after filtration the residue was washed with dichloro-methane and dried. 741 mg (76%) of the title compound **28a - 4** were isolated as solid.

### Part C

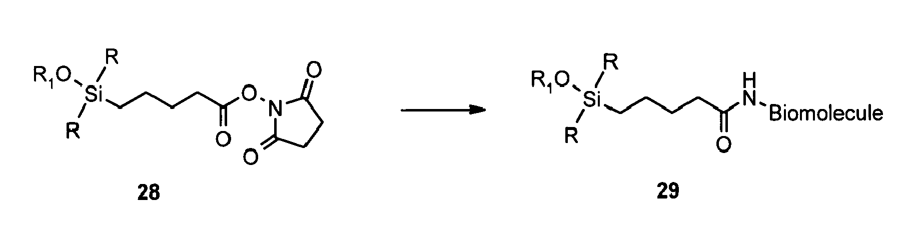

### 5-[Diisobutyl-(4-phenyl-butoxy)-silanyl]-pentanoic acid phenylamide

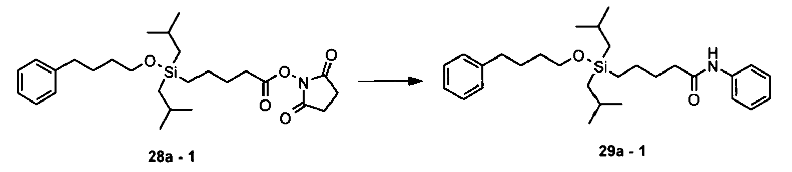

To a solution of 100 mg (204 µmol) 5-[Diisobutyl-(4-phenyl-butoxy)-silanyl]-pentanoic acid 2,5-dioxo-pyrrolidin-1-yl ester **28a - 1** in 2 mL dichloro-methane were added 18.5 µL aniline, 28.3 µL triethylamine and a catalytic amount of dimethyl-pyridin-4-yl-amine. The mixture was stirred at 23°C for 2 days and purified by chromatography to yield 49 mg (51%) of the title compound **29a - 1** were isolated as an oil.
¹H-NMR (CDCI₃): δ = 7.54 (2H), 7.40-7.10 (9H), 3.63 (2H), 2.65 (2H), 2.38 (2H), 1.91-1.29 (8H), 0.98 (14H), 0.69 (2H) 0.63 (4H) ppm.

### Part D

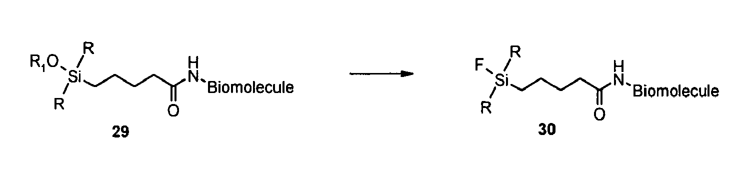

### 5-(Fluoro-diisobutyl-silanyl)-pentanoic acid phenylamide

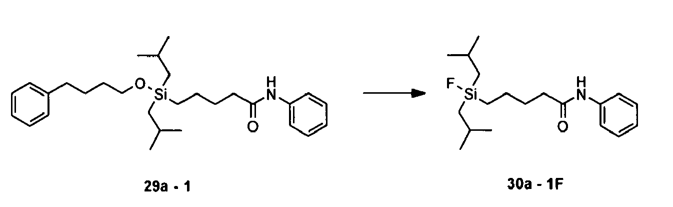

49 mg (105 µmol) 5-[Diisobutyl-(4-phenyl-butoxy)-silanyl]-pentanoic acid phenylamide 29a - 1 was transformed in analogy to silanols or silanes to yield after isolation and purification 22 mg (62%) of the title compound 30a - 1 F as an oil.
¹H-NMR (CDC1₃): δ= 7.54 (2H), 7.35 (2H), 7.17 (1H), 7.13 (1H), 2.40 (2H), 1.90 (2H), 1.81 (2H), 1.53 (2H), 0.99+1.00 (12H), 0.77 (2H), 0.71 (4H) ppm. ¹⁹F-NMR (CDC1₃): δ= -166.9 ppm.

### Radiochemistry

### General Radiolabeling Method which include the use of "organic acid":

¹⁸F-Fluoride was azeotropically dried in the presence of Kryptofix 222 (5mg), potassium carbonate (1mg) in water (500µl) and MeCN (1.5ml) by heating under nitrogen at 100°C for 20-30 minutes. During this time 2-3 × 1ml MeCN were added and evaporated to give the dried Kryptofix 222/K₂CO₃ complex (up to 7.0GBq). After drying, a solution of the precursor (150-300µl of 0.005 - 0.09M in DMSO) and AcOH (3µl) was added. The reaction vessel was heated in the range of RT-90°C for 10-30mins to effect labeling. The crude reaction mixture was analyzed by analytical HPLC.

### [¹⁸F]Fluoro-diisopropyl-{4-[2-(tetrahydro-pyran-2-yloxy)-ethyl]-phenyl}-silane

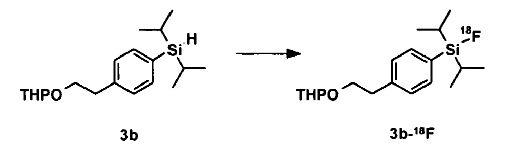

[¹⁸F]Fluoride was eluted from the QMA Light cartridge (Waters) into a Reactivial (10ml) with a solution of Kryptofix 222 (5mg), potassium carbonate (1mg) in water (500µl) and MeCN (1.5ml). The solvent was removed by heating at 100°C under vacuum with a stream of nitrogen. Anhydrous MeCN (1ml) was added and evaporated as before. This step was repeated again to give the dried Kryptofix 222/K₂CO₃ complex (276MBq). A solution of diisopropyl-{4-[2-(tetrahydro-pyran-2-yloxy)-ethyl]-phenyl}-silane 3b (6.4mg) in anhydrous DMSO (300µl) and AcOH (3µl) was added. After heating at 65°C for 15mins an aliquot of the reaction mixture was diluted with MeCN (1ml). The crude reaction mixture was analyzed using an analytical HPLC (Column Hamilton PRP-1, 250x4.1mm, 7µ, 1.2ml/min, solvent MeCN/H₂O 85:15 isocratic 30mins). The yield determined by HPLC was 34%. The peak of the [¹⁸F]-labeled product was confirmed by coninjection.

### 2-[4-([¹⁸F]Fluoro-diisopropyl-silanyl)-phenyl]-ethanol

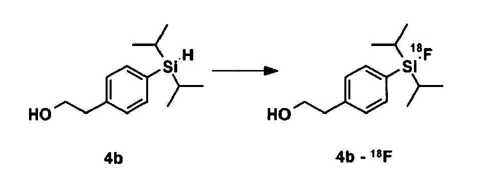

[¹⁸F]Fluoride was eluted from the QMA Light cartridge (Waters) into a Reactivial (10ml) with a solution of Kryptofix 222 (5mg), potassium carbonate (1mg) in water (500µl) and MeCN (1.5ml). The solvent was removed by heating at 100°C under vacuum with a stream of nitrogen. Anhydrous MeCN (1ml) was added and evaporated as before. This step was repeated again to give the dried Kryptofix 222/K₂CO₃ complex (589MBq). A solution of 2-(4-diisopropylsilanyl-phenyl)-ethanol 4b (6.3mg) in anhydrous DMSO (300µl) and AcOH (3µl) was added. After heating at 65°C for 15mins an aliquot of the reaction mixture was diluted with MeCN (1ml). The crude reaction mixture was analyzed using an analytical HPLC (Column Hamilton PRP-1, 250×4.1mm, 7µ, 1.2ml/min, solvent MeCN/H₂O 85:15 isocratic 30mins). The yield determined by HPLC was 70%. The peak of the [¹⁸F]-labeled product was confirmed by coninjection.

### 2-[4-([¹⁸F]Fluoro-diisopropyl-silanyl)-phenyl]-acetyl-Val-βAla-Phe-Gly-NH₂

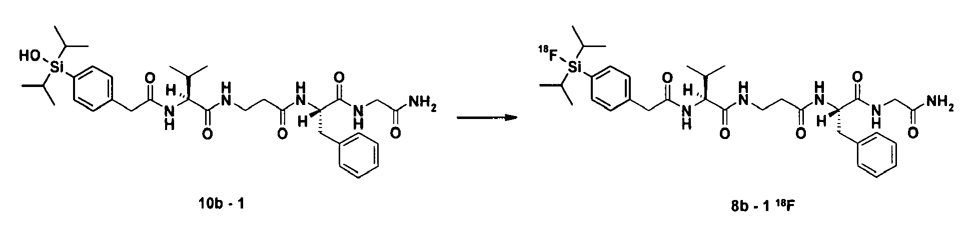

[¹⁸F]Fluoride was eluted from the QMA Light cartridge (Waters) into a Reactivial (10ml) with a solution of Kryptofix 222 (5mg), potassium carbonate (1mg) in water (500µl) and MeCN (1.5ml). The solvent was removed by heating at 100°C under vacuum with a stream of nitrogen. Anhydrous MeCN (1ml) was added and evaporated as before. This step was repeated again to give the dried Kryptofix 222/K₂CO₃ complex (2.0GBq). A solution of 2-[4-(hydroxy-diisopropyl-silanyl)-phenyl]-acetyl-Val-ßAla-Phe-Gly-NH₂ 10b-1 (1.0mg) in anhydrous DMSO (300µl) and AcOH (3µl) was added. After heating at 90°C for 15mins an aliquot of the reaction mixture was diluted with MeCN (1ml). The crude reaction mixture was analyzed using an analytical HPLC (Column Hamilton PRP-1, 250×4.1mm, 7µ, 1.0ml/min, solvent MeCN/H₂O 45:55 isocratic 15mins). The yield determined by HPLC was 53%. The peak of the [¹⁸F]-labeled product was confirmed by coninjection.

### 3-[3-([¹⁸F]Fluoro-diisopropyl-silanyl)-phenyl]-propanyl-Val-βAla-Phe-Gly-NH₂

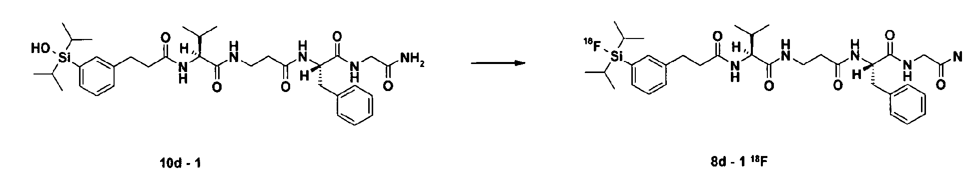

[¹⁸F]Fluoride was eluted from the QMA Light cartridge (Waters) into a Reactivial (10ml) with a solution of Kryptofix 222 (5mg), potassium carbonate (1mg) in water (500µl) and MeCN (1.5ml). The solvent was removed by heating at 100°C under vacuum with a stream of nitrogen. Anhydrous MeCN (1ml) was added and evaporated as before. This step was repeated twice to give the dried Kryptofix 222/K₂CO₃ complex (4.36GBq). A solution of 3-[3-(hydroxy-diisopropyl-silanyl)-phenyl]-propyl-Val-ßAla-Phe-Gly-NH₂ 10d-1 (2.0mg) in anhydrous DMSO (300µl) and AcOH (3µl) was added. After heating at 90°C for 30mins an aliquot of the reaction mixture was diluted with MeCN (1ml). The crude reaction mixture was analyzed using an analytical HPLC (Column Hamilton PRP-1, 250×4.1mm, 7µ, 0.8ml/min, solvent 10mM K₂HPO₄ in H₂O / 10mM K₂HPO₄ in MeCN/H₂O (7:3) 70:30 isocratic 20mins). The yield determined by HPLC was 46%. The peak of the [¹⁸F]-labeled product was confirmed by coninjection.

### Benzyl 2-(di-tert-butyl-[¹⁸F]fluorosilyl)acetate

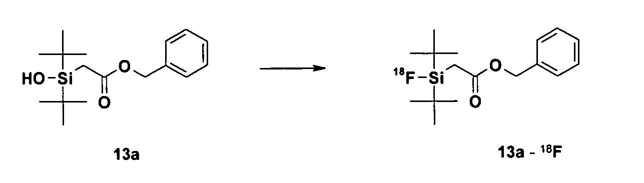

[¹⁸F]Fluoride was eluted from the QMA Light cartridge (Waters) into a Reactivial (10ml) with a solution of Kryptofix 222 (5mg), potassium carbonate (1mg) in water (500µl) and MeCN (1.5ml). The solvent was removed by heating at 100°C under vacuum with a stream of nitrogen. Anhydrous MeCN (1ml) was added and evaporated as before. This step was repeated twice to give the dried Kryptofix 222/K₂CO₃ complex. A solution of benzyl 2-(di-tert-butyl(hydroxy)silyl)acetate 13a (5.0mg) in anhydrous DMSO (300µl) and AcOH (3µl) was added. After 15mins at RT an aliquot of the reaction mixture was diluted with MeCN (1ml). The crude reaction mixture was analyzed using an analytical HPLC (Column Hamilton PRP-1, 250×4.1mm, 7µ, 1.2ml/min, solvent MeCN/H₂O 85:15 isocratic 15mins). The yield determined by HPLC was 79%. The peak of the [¹⁸F]-labeled product was confirmed by coninjection.

### N-(3-([¹⁸F]fluorodimethylsilyl)propyl)biphenyl-4-carboxamide

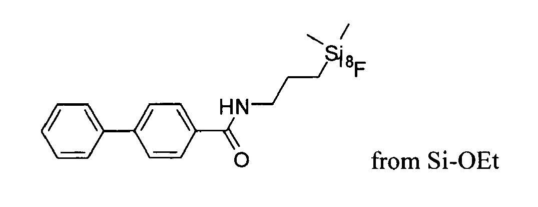

A solution of *N*-(3-(ethoxydimethylsilyl)propyl)biphenyl-4-carboxamide (5.2mg) in anhydrous DMSO (300µl) and AcOH (3µl) was added was added to the dried Kryptofix 222 / K₂CO₃ complex. After heating at 65°C for 10min an aliquot of the reaction mixture was diluted with CH₃CN (1ml). The crude reaction mixture was analyzed using an analytical HPLC (CH₃CN / H₂O 50:50 isocratic, 1ml/min, *t*_{R}(product) = 7.7min). The yield determined by HPLC was quantitative. The peak of the [¹⁸F]-labeled product was confirmed by coninjection.

### N-(3-([¹⁸F]fluorodi-iso-propylsilyl)propyl)biphenyl-4-carboxamide

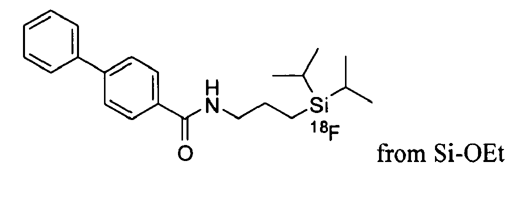

A solution of *N*-(3-(ethoxydi-*iso*-propylsilyl)propyl)biphenyl-4-carboxamide (5.0mg) in anhydrous DMSO (300µl) and AcOH (3µl) was added to the dried Kryptofix 222 / K₂CO₃ complex. After 10min at RT an aliquot of the reaction mixture was diluted with CH₃CN (1ml). The crude reaction mixture was analyzed using an analytical HPLC (CH₃CN / H₂O 87:13 isocratic, 0.8ml/min, *t*_{R}(product) = 12.0min). The yield determined by HPLC was 97%. The peak of the [¹⁸F]-labeled product was confirmed by coninjection.

### [¹⁸F]Fluoro-di-iso-propyl-{4-[2-(tetrahydro-pyran-2-yloxy)-ethyl]-phenyl}-silane from the silanol

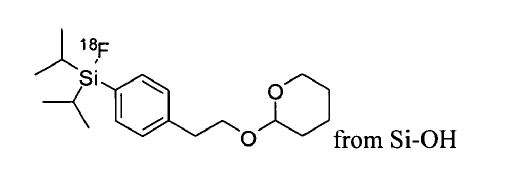

A solution of di-*iso*-propyl-{4-[2-(tetrahydro-pyran-2-yloxy)-ethyl]-phenyl}-silanol (6.1mg) in anhydrous DMSO (300µl) and AcOH (3µl) was added to the dried Kryptofix 222 /K₂CO₃ complex (378MBq). After heating at 65°C for 15min an aliquot of the reaction mixture was diluted with CH₃CN (1ml). The crude reaction mixture was analyzed using an analytical HPLC (CH₃CN / H₂O 85:15 isocratic, 1.2ml/min, *t*_{R}(product) = 16.4min). The yield determined by HPLC was 60%. The peak of the [¹⁸F]-labeled product was confirmed by coninjection.

### [¹⁸F]Fluoro-di-iso-propyl-{4-[2-(tetrahydro-pyran-2-yloxy)-ethyl]-phenyl}-silane from the silane

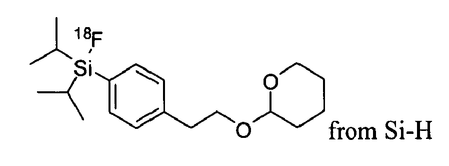

A solution of di-*iso*-propyl-{4-[2-(tetrahydro-pyran-2-yloxy)-ethyl]-phenyl}-silane (5mg) in anhydrous DMSO (300µl) was added to the dried Kryptofix 222 / K₂CO₃ complex (4.3GBq). After heating at 90°C for 30min an aliquot of the reaction mixture was diluted with CH₃CN (1ml). The crude reaction mixture was analyzed using an analytical HPLC (CH₃CN / H₂O 90:10 isocratic, 1.0ml/min, *t*_{R}(product) = 22.3min). The yield determined by HPLC was 97%. The peak of the [¹⁸F]-labeled product was confirmed by coninjection.

### 2-[4-([¹⁸F]Fluoro-di-iso-propyl-silanyl)-phenyl]-ethanol from the silanol

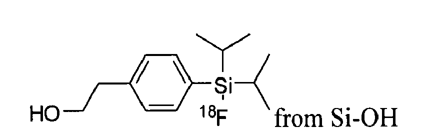

A solution of 2-[4-(hydroxy-di-*iso*-propyl-silanyl)-phenyl]-ethanol (5.1 mg) in anhydrous DMSO (300µl) and AcOH (3µl) was added to the dried Kryptofix 222 / K₂CO₃ complex (364MBq). After heating at 65°C for 15min an aliquot of the reaction mixture was diluted with CH₃CN (1ml). The crude reaction mixture was analyzed using an analytical HPLC (CH₃CN / H₂O 85:15 isocratic, 1.2ml/min, *t*_{R}(product) = 4.1min). The yield determined by HPLC was 86%. The peak of the [¹⁸F]-labeled product was confirmed by coninjection.

### 2-[4-([¹⁸F]Fluoro-di-iso-propyl-silanyl)-phenyl]-ethanol from the silane

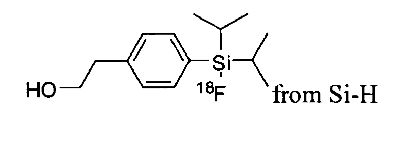

A solution of 2-(4-di-*iso*-propylsilanyl-phenyl)-ethanol (6.3mg) in anhydrous DMSO (300µl) and AcOH (3µl) was added to the dried Kryptofix 222 / K₂CO₃ complex (589MBq). After heating at 65°C for 15min an aliquot of the reaction mixture was diluted with CH₃CN (1ml). The crude reaction mixture was analyzed using an analytical HPLC (CH₃CN / H₂O 85:15 isocratic, 1.2ml/min, *t*_{R}(product) = 4.1min). The yield determined by HPLC was 70%. The peak of the [¹⁸F]-labeled product was confirmed by coninjection.

### 2-[4-([¹⁸F]Fluoro-di-iso-propyl-silanyl)-phenyl]-acetyl-Val-βAla-Phe-Gly-NH₂

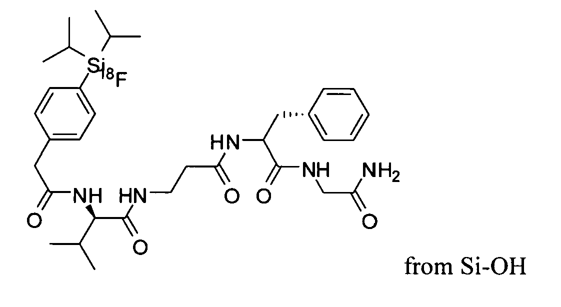

A solution of 2-[4-(hydroxy-di-*iso*-propyl-silanyl)-phenyl]-acetyl-Val-βAla-Phe-Gly-NH₂ (1mg) in anhydrous DMSO (300µl) and AcOH (3µl) was added to the dried Kryptofix 222 / K₂CO₃ complex (2.0GBq). After heating at 90°C for 15min an aliquot of the reaction mixture was diluted with CH₃CN (1ml). The crude reaction mixture was analyzed using an analytical HPLC (CH₃CN / H₂O 45:55 isocratic, 1.0ml/min, *t*_{R}(product) = 12.3min). The yield determined by HPLC was 53%. The peak of the [¹⁸F]-labeled product was confirmed by coninjection.

### 3-[3-([¹⁸F]Fluoro-di-iso-propyl-silanyl)-phenyl]-propanyl-Val-βAla-Phe-Gly-NH₂

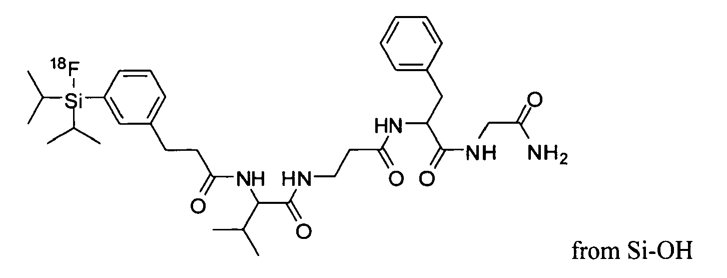

A solution of 3-[3-(hydroxy-di-*iso*-propyl-silanyl)-phenyl]-propyl-Val-βAla-Phe-Gly-NH₂ (2.0mg) in anhydrous DMSO (300µl) and AcOH (3µl) was added to the dried Kryptofix 222 / K₂CO₃ complex (4.36GBq). After heating at 90°C for 30min an aliquot of the reaction mixture was diluted with CH₃CN (1ml). The crude reaction mixture was analyzed using an analytical HPLC (10mM K₂HPO₄ in H₂O / 10mM K₂HPO₄ in CH₃CN / H₂O (7:3) 30:70 isocratic, 0.8ml/min, *t*_{R}(product) = 11.9min). The yield determined by HPLC was 46%. The peak of the [¹⁸F]-labeled product was confirmed by coninjection.

### 2-[4-([¹⁸F]Fluoro-di-iso-propyl-silanyl)-phenyl]-acetyl-Ala-Gln-Trp-Gly-His(3-Me)-FA1010-Leu-NH₂

### (FA01010 is (4R,5S)-4-amino-5-methyl-heptananoic acid incorporated as part of the peptide)

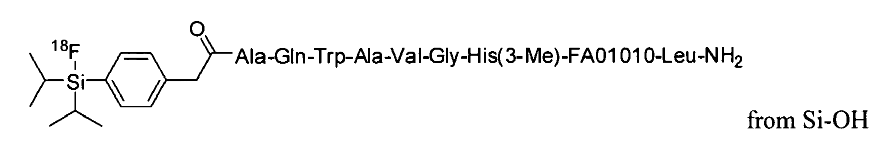

A solution of 3-[3-(hydroxy-di-iso-propyl-silanyl)-phenyl]-propyl-Ala-Gln-Trp-Ala-Val-Gly-His(3-Me)-FA01010-Leu-NH₂ (2.0mg) in anhydrous DMSO (150µl) and AcOH (3µl) was added to the dried Kryptofix 222 / K₂CO₃ complex (7.0GBq). After heating at 90°C for 30min an aliquot of the reaction mixture was diluted with CH₃CN (1ml). The crude reaction mixture was analyzed using an analytical HPLC (10mM K₂HPO₄ in H₂O / 10mM K₂HPO₄ in CH₃CN /H₂O (7:3) 45:55 isocratic, 1.0ml/min, *t*_{R}(product) = 17.2min). The yield determined by HPLC was 34%. The peak of the [¹⁸F]-labeled product was confirmed by coninjection.

### Benzyl 2-(di-tert-butyl-[¹⁸F]fluorosilyl)acetate from the silanol

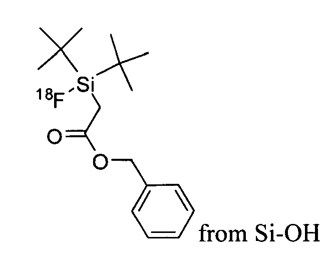

A solution of benzyl 2-(di-*tert*-butyl(hydroxy)silyl)acetate (5.0mg) in anhydrous DMSO (300µl) and AcOH (3µl) was added to the dried Kryptofix 222 / K₂CO₃ complex. After 15min at RT an aliquot of the reaction mixture was diluted with CH₃CN (1ml). The crude reaction mixture was analyzed using an analytical HPLC (CH₃CN / H₂O 85:1 5 isocratic, 1.2ml/min, *t*_{R}(product) = 8.4min). The yield determined by HPLC was 79%. The peak of the [¹⁸F]-labeled product was confirmed by coninjection.

### Benzyl 2-(di-tert-butyl-[¹⁸F]fluorosilyl)acetate from the benzyloxysilane

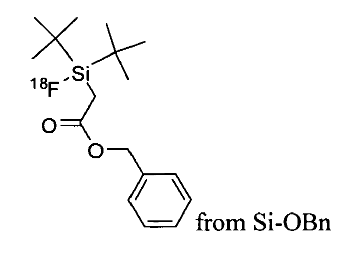

A solution of benzyl 2-(benzyloxydi-tert-butylsilyl)acetate (6.0mg) in anhydrous DMSO (300µl) and AcOH (3µl) was added to the dried Kryptofix 222 / K₂CO₃ complex (3.4GBq). After 15min at RT an aliquot of the reaction mixture was diluted with CH₃CN (1ml). The crude reaction mixture was analyzed using an analytical HPLC (CH₃CN / H₂O 85:15 isocratic, 1.2ml/min, *t*_{R}(product) = 8.7min). The yield determined by HPLC was 28%. The peak of the [¹⁸F]-labeled product was confirmed by coninjection.

### 1-(Di-tert-butylfluorosilyl)acetyl -Val-βAla-Phe-Gly-NH₂

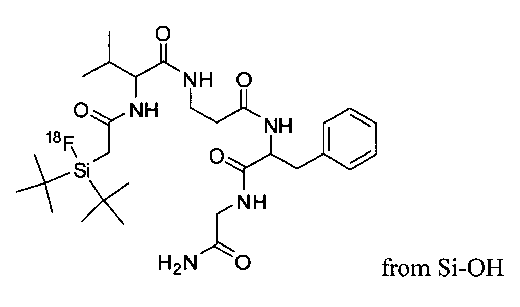

A solution of 2-(di-*tert*-butyl(hydroxy)silyl)acetyl-Val-βAla-Phe-Gly-NH₂ (2mg) in anhydrous DMSO (300µl) and AcOH (3µl) was added to the dried Kryptofix 222 / K₂CO₃ complex. After heating at 50°C for 15min an aliquot of the reaction mixture was diluted with CH₃CN (1ml). The crude reaction mixture was analyzed using an analytical HPLC (10mM K₂HPO₄ in H₂O / 10mM K₂HPO₄ in CH₃CN / H₂O (7:3) 40:60 isocratic, 0.8ml/min, *t*_{R}(product) = 16.6min). The yield determined by HPLC was 82%. The peak of the [¹⁸F]-labeled product was confirmed by coninjection.

### N-Benzyl-2-(4-(di-tert-butyl[¹⁸F]fluorosilyl)phenyl)acetamide from the silane

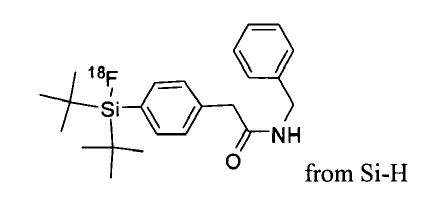

A solution of *N*-benzyl-2-(4-(di-*tert*-butylsilyl)phenyl)acetamide (5mg) in anhydrous DMSO (300µl) was added to the dried Kryptofix 222 / K₂CO₃ complex (2.3GBq). After 15min at 65°C an aliquot of the reaction mixture was diluted with CH₃CN (1ml). The crude reaction mixture was analyzed using an analytical HPLC (CH₃CN / H₂O 80:20 isocratic, 0.8ml/min, *t*_{R}(product) = 10.1min). The yield determined by HPLC was 73%. The peak of the [¹⁸F]-labeled product was confirmed by coninjection.

### N-Benzyl-2-(4-(di-tert-butyl[¹⁸F]fluorosilyl)phenyl)acetamide from the silanol

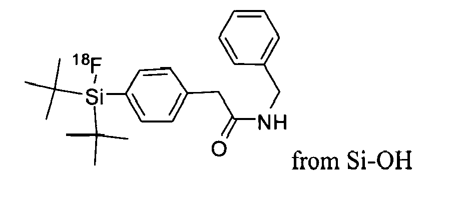

A solution of *N*-benzyl-2-(4-(di-*tert*-butyl(hydroxy)silyl)phenyl)acetamide (5mg) in anhydrous DMSO (300µl) and AcOH (3µl) was added to the dried Kryptofix 222 / K₂CO₃ complex (1.9GBq). After 15min at 65°C an aliquot of the reaction mixture was diluted with CH₃CN (1ml). The crude reaction mixture was analyzed using an analytical HPLC (CH₃CN / H₂O 80:20 isocratic, 0.8ml/min, *t*_{R}(product) = 10.9min). The yield determined by HPLC was 68%. The peak of the [¹⁸F]-labeled product was confirmed by coninjection.

### 2-(4-(Di-tert-butyl[¹⁸F]fluorosilyl)phenyl)acetyl-Ala-Gln-Trp-Gly-His(3-Me)-FA1010-Leu-NH₂

### (FA01010 is (4R,5S)-4-amino-5-methyl-heptananoic acid incorporated as part of the peptide)

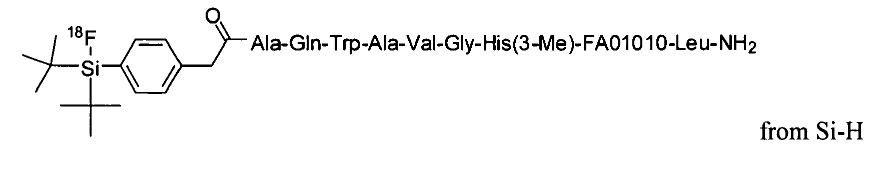

A solution of 2-(4-(di-*tert*-butylsilyl)phenyl)acetyl-Ala-Gln-Trp-Ala-Val-Gly-His(3-Me)-FA01010-Leu-NH₂ (1.5mg) in anhydrous DMSO (150µl) was added to the dried Kryptofix 222 / K₂CO₃ complex (43GBq). After heating at 70°C for 30min an aliquot of the reaction mixture was diluted with CH₃CN (1ml). The crude reaction mixture was analyzed using an analytical HPLC (10mM K₂HPO₄ in H₂O / 10mM K₂HPO₄ in CH₃CN / H₂O (7:3) 30:70 isocratic, 0.8ml/min, *t*_{R}(product) = 8.6min). The yield determined by HPLC was 19%. The peak of the [¹⁸F]-labeled product was confirmed by coninjection.

### Examples for Hydrolytic Stability

The time dependent degree of hydrolysis of compounds of formula I in which X is a fluorine atom of isotope 19 to compounds of formula I in which X is a hydroxy group was determined at physiological pH (7.0). From the kinetics the hydrolytic halflife (t_{1/2}) measured in hours [h] of compounds of formula I in which X is a fluorine atom of isotope 19 were calculated (Table 1, column 2).

The ratio of the hydrolytic halflifes of compounds of formula I in which X is a fluorine atom of isotope 19 and the radioactive halflife of the 18-F isotope (1.83 hours) is defined as relative stability (Table 1, column 3).

After 6 hours 90% of the 18-F isotope have been decayed. The fraction of intact compounds of formula I in which X is a fluorine atom of isotope 19 in % is given in Table 1, column 4.

Compounds of formula I in which X is a fluorine atom of isotope 18 are considered as useful for PET applications if the relative stability of their analogs bearing a fluorine atom of isotope 19 exceed a factor of four and if at least 50% of these compounds remain intact after 6 hours under hydrolytic conditions at pH 7.

Data are listed in Table 1

**Table 1**

| Compound | | t_{1/2} [h] | rel. stability | % intact after 6h |
|---|---|---|---|---|
| 5b - F | | 24.7 | 13.5 | 82 |
| 4b - F | | 66.8 | 36.5 | 84 |
| 3b - F | | 76.4 | 41.8 | 94 |
| 3a - F | | 49.4 | 27.0 | 90 |
| 5a - F | | 10.9 | 6.1 | 66 |
| 4a - F | | 28.1 | 15.4 | 85 |
| 3c - F | | 99.9 | 54.6 | 95 |
| 4c - F | | > 100.0 | > 54.6 | 90 |
| 5c - F | | 59.0 | 32.2 | 92 |
| 3d - F | | > 100.0 | > 54.6 | > 95 |
| 4d - F | | 43.3 | 23.7 | 89 |
| 5d - F | | 22.2 | 12.1 | 81 |
| 3e - F | | > 100.0 | > 54.6 | > 95 |
| 4e - F | | 23.3 | 12.7 | 78 |
| 5e - F | | 20.0 | 10.9 | 78 |
| 5h-F | | 8.7 | 4.8 | 61 |
| 3h-F | | 19.2 | 10.7 | 79 |
| 3g-F | | 8.1 | 4.5 | 59 |
| 5g-F | | 3.5 | 1.9 | 31 |
| 3f-F | | 0.07 | 0.04 | << 1 |
| 22-F | | 33.6 | 18.4 | 83 |
| 30a - 1F | | 13.8 | 7.5 | 71 |

## Claims

1. Compound of general formula I 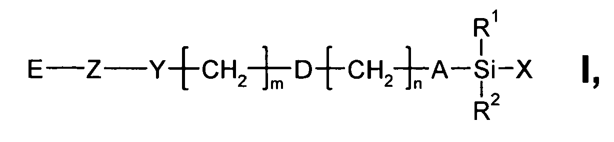 wherein
X represents a leaving group suitable for fluorination, selected from hydrogen, or OR³,
wherein R³ represents hydrogen, C₂-C₁₀ alkyl, C₂-C₁₀ alkenyl, C₂-C₁₀ alkynyl, aryl, heteroaryl, or aralkyl;
R¹ and R², independently, represent hydrogen, C₁-C₁₀ alkyl, aryl, heteroaryl, or aralkyl;
A represents a bond, or aryl, substituted aryl or heteroaryl;
m and n, independently, can be any integer between 0 to 5;
D represents a bond, a sulphur atom, an oxygen atom, or NR⁴,
wherein R⁴ represents hydrogen, C₁-C₁₀ alkyl, aryl, heteroaryl, or aralkyl;
E-Z-Y represents a moiety selected from EO-C(=O), ENR⁵-C(=O), EC(=O)-O, EC(=O)-NR⁵, ENR⁵-SO₂, ESO₂-NR⁵, E-O, E-(S)p, E-NR⁵, ENR⁶C(=O)-NR⁷, ENR⁶-C(=O)NR⁷, ENR⁶C(=S)-NR⁷, ENR⁶-C(=S)NR⁷, EO-C(=O)O, EOC(=O)-O, EOC(=S)-O, EO-C(=S)O (wherein the single bond explicitly shown in the formulae is the bond between Z and Y) and 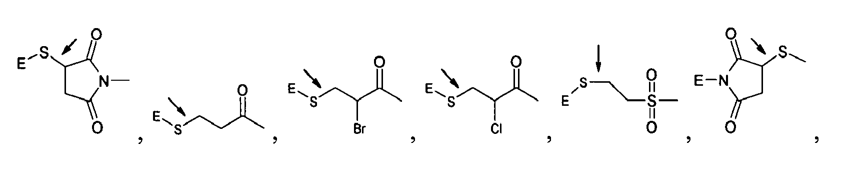 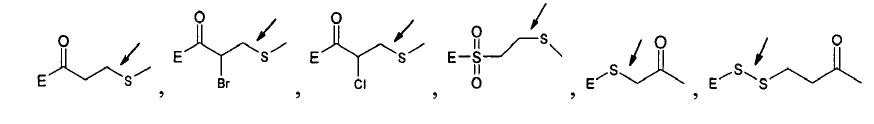 (wherein the arrow indicates the bond between Z and Y),
wherein R⁵, R⁶ and R⁷, independently, represent hydrogen, C₁-C₁₀ alkyl, aryl, heteroaryl, or aralkyl, and
p can be any integer between 1 and 3; and
E-Z is a targeting agent radical.

2. Compound according to claim 1, wherein the leaving group X is selected from the group consisting of (C₆-C₂₀) alkoxy, (C₆-C₁₀) alkynyloxy, or ar(C₆-C₂₀)alkoxy.

3. Compound according to claim 1, wherein the leaving group X is selected from the group consisting of hydrogen and hydroxy.

4. Compound according to claim 1 or 2, wherein R¹ and R², independently, are branched C₂-C₅ alkyl groups.

5. Compound according to claim 4, wherein R¹ and R² are isopropyl, tert-butyl or isobutyl.

6. Compound according to any of claims 1 to 5, wherein the targeting agent is selected from peptides, small molecules or oligonucleotides.

7. Compound according to any of claims 1 to 6, wherein is the targeting agent is -NR⁴-peptide or -NR⁴-(CH₂)ₙ- peptide, R⁴ being independently selected from hydrogen or alkyl, wherein n is between 1 and 6.

8. Compound according to claim 6 or 7, wherein the targeting agent is a peptide comprising from 4 to 100 amino acids.

9. Compound of general formula II 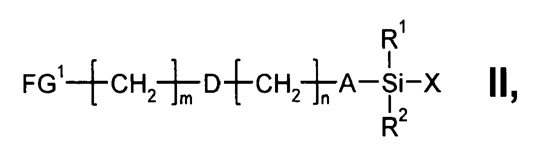 wherein
FG¹ represents OH, Hal, N₃, CO₂R⁸, NHR⁵, N=C=O, N=C=S, O-SO₂-Aryl, OSO₂-Alkyl, SO₂-Hal, S₃H, SH, O-C(=O)-Hal, O-C(=S)-Hal, 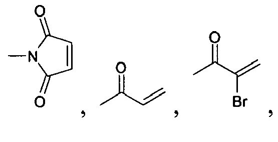 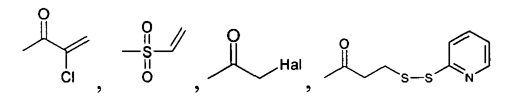 wherein Hal represents a halogen atom, and
R⁸ represents hydrogen, C₁-C₁₀ alkyl, C₂-C₁₀ alkenyl, aralkyl, or 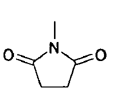 and wherein X, R¹, R², A, D, m and n have the same meanings as in formula I.

10. Compound according to claim 9, wherein the compound is
(Di-tert-butyl-hydroxy-silanyl)-acetic acid
[4-(Hydroxy-diisopropyl-silanyl)-phenyl]-acetic acid
[4-(Hydroxy-diisopropyl-silanyl)-phenyl]-acetic acid
(4-Diisopropylsilanyl-phenyl)-acetic acid
(4-Diisopropylsilanyl-phenyl)-acetic acid 2,5-dioxo-pyrrolidin-1-yl ester [4-(Hydroxy-diisopropyl-silanyl)-phenyl]-acetic acid 2,5-dioxo-pyrrolidin-1-yl ester 4-Diisopropylsilanyl-benzoic acid
4-(Hydroxy-diisopropyl-silanyl)-benzoic acid
4-Diisopropylsilanyl-benzoic acid 2,5-dioxo-pyrrolidin-1-yl ester
4-(Hydroxy-diisopropyl-silanyl)-benzoic acid 2,5-dioxo-pyrrolidin-1-yl ester
3-(4-Diisopropylsilanyl-phenyl)-propionic acid
3-(4-Diisopropylsilanyl-phenyl)-propionic acid 2,5-dioxo-pyrrolidin-1-yl ester
3-(3-Diisopropylsilanyl-phenyl)-propionic acid
3-(3-Diisopropylsilanyl-phenyl)-propionic acid 2,5-dioxo-pyrrolidin-1-yl ester
(3-Diisopropylsilanyl-phenyl)-acetic acid
(3-Diisopropylsilanyl-phenyl)-acetic acid 2,5-dioxo-pyrrolidin-1-yl ester
(4-Diisobutylsilanyl-phenyl)-acetic acid
(4-Diisobutylsilanyl-phenyl)-acetic acid 2,5-dioxo-pyrrolidin-1-yl ester
[4-(Hydroxy-diisobutyl-silanyl)-phenyl]-acetic acid 2,5-dioxo-pyrrolidin-1-yl ester
[4-(Hydroxy-diisobutyl-silanyl)-phenyl]-acetic acid
4-Diisobutylsilanyl-benzoic acid
4-(Hydroxy-diisobutyl-silanyl)-benzoic acid
4-Diisobutylsilanyl-benzoic acid 2,5-dioxo-pyrrolidin-1-yl ester
4-(Hydroxy-diisobutyl-silanyl)-benzoic acid 2,5-dioxo-pyrrolidin-1-yl ester
4-[3-(Ethoxy-diisopropyl-silanyl)-propylcarbamoyl]-butyric acid
4-[3-(Ethoxy-diisopropyl-silanyl)-propylcarbamoyl]-butyric acid 2,5-dioxo-pyrrolidin-1-yl ester
5-[Diisobutyl-(4-phenyl-butoxy)-silanyl]-pentanoic acid 2,5-dioxo-pyrrolidin-1-yl ester
5-[(4-Polystyrene-methoxy-benzyloxy)-diisobutyl-silanyl]-pentanoic acid 2,5-dioxo-pyrrolidin-1-yl ester
5-(Polystyrene-methoxy-diisobutyl-silanyl)-pentanoic acid 2,5-dioxo-pyrrolidin-1-yl ester
or
5-(Polystyrene-ethoxy-diisobutyl-silanyl)-pentanoic acid 2,5-dioxo-pyrrolidin-1-yl ester

11. Method for producing a compound according to general formula I, as defined in claim 1, wherein a compound of general formula II, as defined in claim 9, is reacted with a compound of general formula III
E-FG² III
wherein
FG² has the meanings as listed for FG¹, and E has the same meaning as defined in claim 1, and wherein FG¹ and FG² are selected to establish Z-Y, as defined in claim 1.

12. Method for producing a compound of general formula I, as defined in claim 1 - 8, or of general formula II, as defined in claim 9, wherein X is ¹⁸F, by reacting a compound of general formula I or II, respectively, with a ¹⁸F fluorination agent under appropriate reaction conditions.

13. Method according to claim 12, wherein the reaction temperature is 80°C or less.

14. Method according to claim 13, wherein the reaction temperature is 50°C or less.

15. Compound obtainable according to any of claims 11 to 14, wherein the compound is
[4-(Fluoro-diisopropyl-silanyl)-phenyl]-acetic acid
2-[4-(Fluoro-diisopropyl-silanyl)-phenyl]-ethanol
4-(Fluoro-diisopropyl-silanyl)-benzoic acid
[4-(Fluoro-diisopropyl-silanyl)-phenyl]-methanol
3-[4-(Fluoro-diisopropyl-silanyl)-phenyl]-propan-1-ol
3-[4-(Fluoro-diisopropyl-silanyl)-phenyl]-propionic acid
3-[3-(Fluoro-diisopropyl-silanyl)-phenyl]-propan-1-ol
3-[3-(Fluoro-diisopropyl-silanyl)-phenyl]-propionic acid
2-[3-(Fluoro-diisopropyl-silanyl)-phenyl]-ethanol
[3-(Fluoro-diisopropyl-silanyl)-phenyl]-acetic acid
[4-(Fluoro-diisobutyl-silanyl)-phenyl]-acetic acid
or
4-(Fluoro-diisobutyl-silanyl)-benzoic acid
wherein fluoro means ¹⁸F.

16. Composition comprising a compound according to any of claims 1 to 8 or prepared according to claims 12 to 14.

17. Composition according to claim 16, further comprising a physiologically acceptable diluent or carrier.

18. Kit comprising a compound of any of claims 1 to 8 or prepared according to claims 12 to 14 or a composition of claim 16 or 17, in powder form, and a container containing an appropriate solvent for preparing a solution of the compound or composition for administration to an animal, including a human.

19. Use of a compound according to any of claims 1 to 8 or prepared according to claims 12 to 14, of a composition of claim 16 or 17, or of a kit of claim 18 for diagnostic imaging.

20. Use according to claim 19 for positron emission tomography.

21. Use according to claim 19 or 20 for imaging of tumors, imaging of inflammatory and/or neurodegenerative diseases, such as multiple sclerosis or Alzheimer's disease, or imaging of angiogenesis-associated diseases, such as growth of solid tumors, and rheumatoid arthritis.

22. Compound selected from
*N*-(3-([¹⁸F]fluorodimethylsilyl)propyl)biphenyl-4-carboxamide;
*N*-(3-([¹⁸F]fluorodi-*iso*-propylsilyl)propyl)biphenyl-4-carboxamide;
[¹⁸F]Fluoro-di-*iso*-propyl-{4-[2-(tetrahydro-pyran-2-yloxy)-ethyl]-phenyl}-silane;
[¹⁸F]Fluoro-di-*iso*-propyl-{4-[2-(tetrahydro-pyran-2-yloxy)-ethyl]-phenyl}-silane;
2-[4-([¹⁸F]Fluoro-di-*iso*-propyl-silanyl)-phenyl]-ethanol;
2-[4-([¹⁸F]Fluoro-di-*iso*-propyl-silanyl)-phenyl]-ethanol;
2-[4-([¹⁸F]Fluoro-di-*iso*-propyl-silanyl)-phenyl]-acetyl-Val-βAla-Phe-Gly-NH₂;
3-[3-([¹⁸F]Fluoro-di-*iso*-propyl-sitanyl)-phenyl]-propanyl-Val-βAla-Phe-Gly-NH₂;
2-[4-([¹⁸F]Fluoro-di-*iso*-propyl-silanyl)-phenyl]-acetyl-Ala-Gln-Trp-Gly-His(3-Me)-FA1010-Leu-NH₂;
Benzyl 2-(di-*tert*-butyl-[¹⁸F]fluorosilyl)acetate;
Benzyl 2-(di-*tert*-butyl-[¹⁸F]fluorosilyl)acetate;
1-(Di-*tert*-butylfluorosilyl)acetyl -Val-βAla-Phe-Gly-NH_{2;}
*N*-Benzyl-2-(4-(di-*tert*-butyl[¹⁸F]fluorosilyl)phenyl)acetamide;
*N*-Benzyl-2-(4-(di-*tert*-butyl[¹⁸F]fluorosilyl)phenyl)acetamide;
2-(4-(Di-*tert*-butyl[¹⁸F]fluorosilyl)phenyl)acetyl-Ala-Gln-Trp-Gly-His(3-Me)-FA1010-Leu-NH₂.
